# EUROPEAN PATENT SPECIFICATION

(11) **EP 1 312 923 B1**
(45) Date of publication and mention of the grant of the patent: **28.03.2007**
(21) Application number: 02079785.8
(22) Date of filing: 15.11.2002
(51) Int. Cl.: G01N 33/531, C07K 16/44, G01N 33/94, C07D 211/58

(54) **Method and kit for detecting, or determining the quantity of, metabolites of fentanyl and metabolites of fentanyl analogs**
Verfahren und Kit zum Nachweis, oder zum Quantifizieren von, Metaboliten von Fentanyl und Metaboliten von Fentanyl Analoga
Méthode et trousse pour détecter, ou quantifier, des métabolites de fentanyl et des métabolites de fentanyl analogues

(30) Priority: 16.11.2001 EP 01204401
(43) Date of publication of application: 21.05.2003
(73) Proprietor: Randox Laboratories Ltd., Crumlin, County Antrim BT29 4QY (GB)
(72) Inventor: McConnell, Robert Ivan, c/o Randox Laboraties Ltd., County Antrim BT29 4QY, Northern Ireland (GB); Benchikh, El Ouard, c/o Randox Laboraties Ltd., County Antrim BT29 4QY, Northern Ireland (GB); Fitzgerald, Stephen Peter, c/o Randox Lab. Ltd., County Antrim BT29 4QY, Northern Ireland (GB); Lamont, John Victor, c/o Randox Laboraties Ltd., County Antrim BT29 4QY, Northern Ireland (GB)
(74) Representative: O'Connell, Maura

(56) References cited:
- WO-A-96/39425
- ESSAWI MYH AND PORTOGHESE PS: "Synthesis and evaluation of 1- and 2-substituted fentanyl analogues for opioid activity" JOURNAL OF MEDICINAL CHEMISTRY, vol. 26, 1983, pages 348-352, XP002244810
- FELDMAN P L ET AL: "Design, synthesis, and pharmacological evaluation of ultrashort- to long-acting opioid analgeTics" JOURNAL OF MEDICINAL CHEMISTRY, AMERICAN CHEMICAL SOCIETY. WASHINGTON, US, vol. 34, 1991, pages 2202-2208, XP002229431 ISSN: 0022-2623
- LOBBEZOO M W ET AL: "Structure and receptor interactions of morphinomimetics. Part I. Hydroxy and methoxy derivatives of fentanyl and some morphine analogUES" EUROPEAN JOURNAL OF MEDICINAL CHEMISTRY, EDITIONS SCIENTIFIQUE ELSEVIER, PARIS, FR, no. 4, July 1980 (1980-07), pages 357-361, XP002216852 ISSN: 0223-5234
- BURKE T R ET AL: "Probes for narcotic receptor mediated phenomena. 7. Synthesis and pharmacological properties of irreversible ligands specific for.mu. or.delta. opiate receptors" JOURNAL OF MEDICINAL CHEMISTRY, AMERICAN CHEMICAL SOCIETY. WASHINGTON, US, vol. 27, 1984, pages 1570-1574, XP002216853 ISSN: 0022-2623
- MICHIELS M ET AL: "A SENSITIVE RADIOIMMUNOASSAY FOR FENTANYL PLASMA LEVEL IN DOGS AND MAN" EUROPEAN JOURNAL OF CLINICAL PHARMACOLOGY, SPRINGER VERLAG, DE, vol. 2, no. 12, 14 October 1977 (1977-10-14), pages 153-158, XP008004998 ISSN: 0031-6970
- WOODS, W. E. ET AL: "High-sensitivity radioimmunoassay screening method for fentanyl" AM. J. VET. RES., vol. 47, no. 10, 1986, pages 2180-2183, XP008004948
- MARYANOFF B E ET AL: "Potential affinity labels for the opiate receptor based on fentanyl and related compounds." JOURNAL OF MEDICINAL CHEMISTRY. UNITED STATES AUG 1982, vol. 25, no. 8, August 1982 (1982-08), pages 913-919, XP002206128 ISSN: 0022-2623
- FRINCKE, JAMES M. ET AL: "The major metabolite of fentanyl in the horse" DRUG METAB. DISPOS. (1980), 8(6), 425-7, 1980, XP008004950
- DATABASE MEDLINE [Online] March 1993 (1993-03) SILVERSTEIN J H ET AL: "An analysis of the duration of fentanyl and its metabolites in urine and saliva." Database accession no. NLM8452277 XP002206130 & ANESTHESIA AND ANALGESIA. UNITED STATES MAR 1993, vol. 76, no. 3, March 1993 (1993-03), pages 618-621, ISSN: 0003-2999
- WATTS V W ET AL: "EVALUATION OF THE COAT-A-COUNT IODINE-125 FENTANYL RIA COMPARISON OF IODINE-125 RIA AND GC-MS-SIM FOR QUANTIFICATION OF FENTANYL IN CASE URINE SPECIMENS" JOURNAL OF ANALYTICAL TOXICOLOGY, vol. 14, no. 5, 1990, pages 266-272, XP008004883 ISSN: 0146-4760
- DATABASE BIOSIS [Online] BIOSCIENCES INFORMATION SERVICE, PHILADELPHIA, PA, US; 1995 MAKOWSKI GREGORY S ET AL: "An enzyme-linked immunosorbent assay for urinary screening of fentanyl citrate abuse." Database accession no. PREV199598241759 XP002206131 & ANNALS OF CLINICAL AND LABORATORY SCIENCE, vol. 25, no. 2, 1995, pages 169-178, ISSN: 0091-7370
- DATABASE MEDLINE [Online] February 1983 (1983-02) MICHIELS M ET AL: "Radioimmunoassay of the new opiate analgesics alfentanil and sufentanil. Preliminary pharmacokinetic profile in man." Database accession no. NLM6131992 XP002206132 & THE JOURNAL OF PHARMACY AND PHARMACOLOGY. ENGLAND FEB 1983, vol. 35, no. 2, February 1983 (1983-02), pages 86-93, ISSN: 0022-3573
- HENDERSON G L ET AL: "Antibodies to fentanyl." THE JOURNAL OF PHARMACOLOGY AND EXPERIMENTAL THERAPEUTICS. UNITED STATES FEB 1975, vol. 192, no. 2, February 1975 (1975-02), pages 489-496, XP008004880 ISSN: 0022-3565
- HENDERSON, G. L. ET AL: "Metabolism and disposition of fentanyl in man and the horse" PROC. WEST. PHARMACOL. SOC. (1981), 24TH, 137-40, 1981, XP008004949
- MCDONALD J ET AL: "IMMUNOASSAY DETECTION OF DRUGS IN HORSES I. PARTICLE CONCENTRATION FLUOROIMMUNOASSAY DETECTION OF FENTANYL AND ITS CONGENERS" RESEARCH COMMUNICATIONS IN CHEMICAL PATHOLOGY AND PHARMACOLOGY, PJD PUBLICATIONS LTD., WESTBURY, NY, US, vol. 3, no. 57, September 1987 (1987-09), pages 389-407, XP008004997 ISSN: 0034-5164
- RUANGYUTTIKARN W ET AL: "Detection of fentanyl and its analogs by enzyme-linked immunosorbent assay." JOURNAL OF ANALYTICAL TOXICOLOGY. UNITED STATES 1990 MAY-JUN, vol. 14, no. 3, May 1990 (1990-05), pages 160-164, XP008004878 ISSN: 0146-4760
- HAMMARGREN W R ET AL: "ANALYZING NORMETABOLITES OF THE FENTANYLS BY GAS CHROMATOGRAPHY-ELECTRON CAPTURE DETECTION" JOURNAL OF ANALYTICAL TOXICOLOGY, vol. 12, no. 4, 1988, pages 183-191, XP008004879 ISSN: 0146-4760
- CHAPPEY O N ET AL: "MONOCLONAL ANTIBODIES IN HAPTEN IMMUNOASSAYS" PHARMACEUTICAL RESEARCH, NEW YORK, NY, US, vol. 11, no. 9, November 1992 (1992-11), pages 1375-1379, XP008005000 ISSN: 0724-8741
- ROSNER M H ET AL: "IMMUNOCHEMICAL TECHNIQUES IN BIOLOGICAL MONITORING" ENVIRONMENTAL HEALTH PERSPECTIVES,, no. 94, August 1991 (1991-08), pages 131-134, XP008005001 ISSN: 0091-6765

## Description

The present invention relates to a method and kit for detecting, or determining the quantity of, metabolites, preferably nor-metabolites, both of fentanyl and of its analogs, as well as, immunogens, conjugates and antibodies useful therein.

By "detecting" is meant qualitatively analysing for the presence or absence of a substance.

By "determining" is meant quantitatively analysing for the amount of a substance present.

By "fentanyl" is meant N-(1-phenethyl-4-piperidinyl)-N-phenylpropionamide.

By "fentanyl analogs" is meant substances sharing structural similarity with the N-phenyl-N-(4-piperidinyl) amine structure of fentanyl and sharing pharmacological activity with fentanyl. Such "fentanyl analogs" include, but are not limited to, sufentanil, carfentanil, acetylfentanyl, *p*-fluorofentanyl, benzylfentanyl, thienylfentanyl, α-methylthienylfentanyl, butyrylfentanyl and alfentanil as well as, the so-called designer drugs, ∝-and *p*-methylfentanyl and 3-cis/trans-methylfentanyl.

By "metabolites" is meant the breakdown products, *in vivo*, both of fentanyl and of fentanyl analogs. Thus, for fentanyl, this embraces nor-fentanyl (N-(propionyl)-4-N-anilinopiperidine), despropionyl fentanyl (N-(1-phenethyl-4-piperidinyl)-phenylamine), *p*-hydroxyfentanyl (N-(*p*-hydroxyphenyl)-N-[1-(2-phenylethyl)-4-piperidinyl] propanamide) and *p*-hydroxy nor-fentanyl (N-propionyl-4-N-(*p*-hydroxyanilino) piperidine).

The present invention is intended to have broad applicability across metabolites, both of fentanyl and of fentanyl analogs. The present invention is intended to have particular applicability to nor-metabolites, both of fentanyl and of fentanyl analogs. The present invention is intended to have most particular applicability to nor-metabolites of fentanyl. The present invention is also intended to have broad applicability across fentanyl itself and its analogs.

Fentanyl is a powerful synthetic opioid with at least 80 times the potency of morphine as an analgesic. It is routinely used peri-operatively for the induction and maintenance of anaesthesia and postoperatively for analgesia. Analogs of fentanyl have been synthesised for increasing its analgesic and euphoric effect. Due to their high potency and fast-acting narcotic analgesia, fentanyl and its analogs have abuse potential. Fentanyl distributes rapidly from blood to body tissues and is extensively metabolized. In man, approximately 80% of a fentanyl dose is excreted in urine within 72 hours, with 92% to 98% of this consisting of metabolites. Oxidative N-dealkylation to form nor-fentanyl is the major metabolic pathway for fentanyl in man (Silverstein et al, 1993). N-dealkylation also seems characteristic of fentanyl analogs such as alfentanil and sufentanil (Camu et al (1982) and Meuldermans et al (1982)). In contrast, in horses, the major metabolic pathway is via metabolic oxidation of the propionyl side chain of fentanyl, to form a malonanilinic acid (Frincke et al (1980)). The chemical structure of fentanyl is represented below:

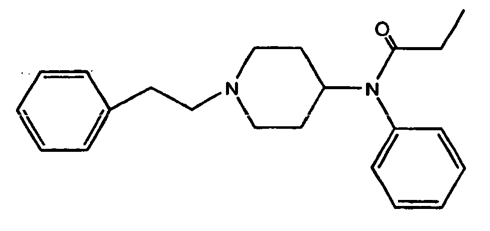

Fentanyl, which has a half life of 10 minutes, is metabolized, at least in humans, to yield mainly:

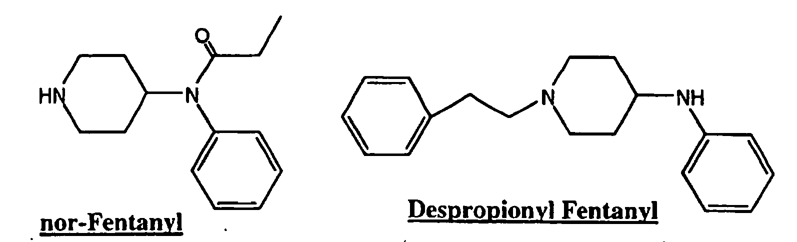

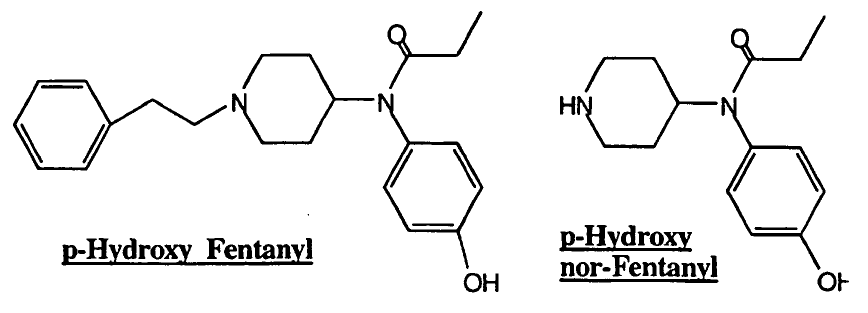

Various methods have been used to determine the quantity of metabolites of fentanyl, and its analogs in a sample. Analytical methods include gas chromatography with flame ionisation, electron capture, and nitrogen sensitive detection, and gas chromatography/mass spectrometry (GC/MS). For example, a sensitive gas-liquid chromatographic method for analyzing the nor-metabolites of fentanyl and 3-methylfentanyl in urine is disclosed in W.R. Hammargren and G.L. Henderson, 1988. The method disclosed includes detection by electron capture detector or mass spectroscopy. Such chromatographic methods are, however, too costly and time consuming for use as screening tools.

Radioimmunoassays used for the determination of the quantity of fentanyl and its analogs are very sensitive, but do require radionuclide tracers, for example ¹²⁵I and ³H, and in some cases, a preliminary extraction step. For example, various different types of radioimmunoassays (RIAs), including solid phase RIA, are used to detect fentanyl and related analogs. Hammargren & Henderson (1988) disclose use of a ¹²⁵I RIA produced by Diagnostic Products. However, it was found that the normetabolites and despropionylmetabolites do not cross-react significantly using the radioimmunoassay. In addition, Watts & Caplan (1990) disclose a fentanyl RIA (also a ¹²⁵I RIA from Diagnostic Products), which confirms the data of Hammargren & Henderson (1988), namely, with poor cross-reactivity for certain fentanyl metabolites, specifically less than 5% (Table IV response analog/response fentanyl) for norfentanyl and 14-30% (Table IV response analog/response fentanyl) for 2-hydroxyfentanyl. Concerning 2-hydroxyfentanyl, it is currently believed that this is not a major metabolite of fentanyl, at least in man, despite the reference thereto in Watts & Caplan. Michiels et al (1977) disclose a ³H RIA for fentanyl from Janssen using carboxyfentanyl as the hapten - no cross-reaction was observed with despropionyl fentanyl and nor-fentanyl, the major metabolites of fentanyl in humans. Similarly, Henderson et al (1975) disclose a fentanyl RIA from McNeil Laboratories using carboxyfentanyl as the hapten but all the nor- and despropionyl- metabolites of fentanyl do not cross-react significantly.

Enzyme-linked immunosorbent assays (ELISAs) are a nonradioactive alternative, which are also known for the determination of the quantity of fentanyl, and its analogs. For example, in Werawan Ruangyuttikarn et al, 1990, a prototype ELISA is disclosed for detecting the presence of fentanyl using a carboxyfentanyl hapten. This disclosure, whilst explicitly silent on cross-reactivity against metabolites of fentanyl, teaches that modification about the piperidine moiety dramatically alters cross-reactivity - this suggests that poor cross-reactivity could be expected for nor-fentanyl. Gregory S. Makowski et al, 1995 discloses an ELISA with poor cross reactivity against despropionyl fentanyl and nor-fentanyl metabolites, specifically, only 0.53% cross-reactivity against despropionyl fentanyl and less than 0.03% cross-reactivity against nor-fentanyl.

Previously reported work on fentanyl immunoassays (whether RIA or ELISA) has solely centred, to the knowledge of the inventors, on derivatization of fentanyl (by a carboxyl group) through the free end of the propionamide group of fentanyl. Such a derivatisation, using -COOH as the crosslinker, results in carboxyfentanyl (N-phenyl-N-[1-(2-phenethyl)-4-piperidinyl] carboxypropanamide) as the hapten, which hapten is disclosed in J. McDonald et al, 1987. J. McDonald et al disclose a particle concentration fluorescence immunoassay (PCFIA) using an antiserum raised from a carboxyfentanyl-bovine serum albumin (BSA) immunogen and carboxyfentanyl linked to b-Phycoerythrin. Similarly, M Michiels et al (1977) disclose a carboxyfentanyl-BSA immunogen. Antibodies raised to a carboxyfentanyl-bovine gamma globulin (BGG) immunogen are disclosed in GL Henderson et al, 1975. However, antibodies and conjugates raised against carboxyfentanyl as the hapten suffer from the disadvantage that there is little or no ability to detect metabolites of fentanyl and/or metabolites of fentanyl analogs.

The present invention describes bonding of a novel hapten derivative of fentanyl or norfentanyl, the hapten being covalently linked either to an antigenicity-conferring carrier material, in order to produce an immunogen, or to a detectable labelling agent, in order to produce a conjugate (or detection reagent). The present invention also describes how antibodies generated to this immunogen are employed in the development of a generic assay, which can be used to determine the quantity of metabolites of fentanyl, and metabolites of its analogs, in fluids, preferably biological fluids, more preferably in biological fluids from humans.

### Objects of the Invention

It is an object of the invention to overcome some or all of the disadvantages of the prior art, or to provide an alternative thereto.

It is an object of a preferred embodiment of the invention to provide a method and a kit for detecting, or determining the quantity of, metabolites of fentanyl and/or metabolites of fentanyl analogs. It is an object of a more preferred embodiment of the invention to provide a method and a kit for detecting, or determining the quantity of, metabolites of fentanyl and/or metabolites of fentanyl analogs, showing more than 5%, preferably more than 25%, cross-reactivity for the metabolites of fentanyl and/or the metabolites of fentanyl analogs, more preferably the nor-metabolites, when compared to fentanyl itself.

It is a further object of a preferred embodiment of the present invention to develop antibodies capable of binding with, as a structural epitope, an N-phenyl-N-(4-piperidinyl) amine moiety.

### Detailed Description of Invention

The invention provides methods, kits, conjugates, immunogens, antibodies and processes as detailed in the appended claims.

In a first aspect, an immunogen is provided comprising a hapten coupled to an antigenicity-conferring carrier material, the hapten being derivatised with a crosslinker at position 1, 2 or 4, preferably at position 1 or 2, with the proviso that the crosslinker is not carboxyl when the hapten is derivatised at position 1.

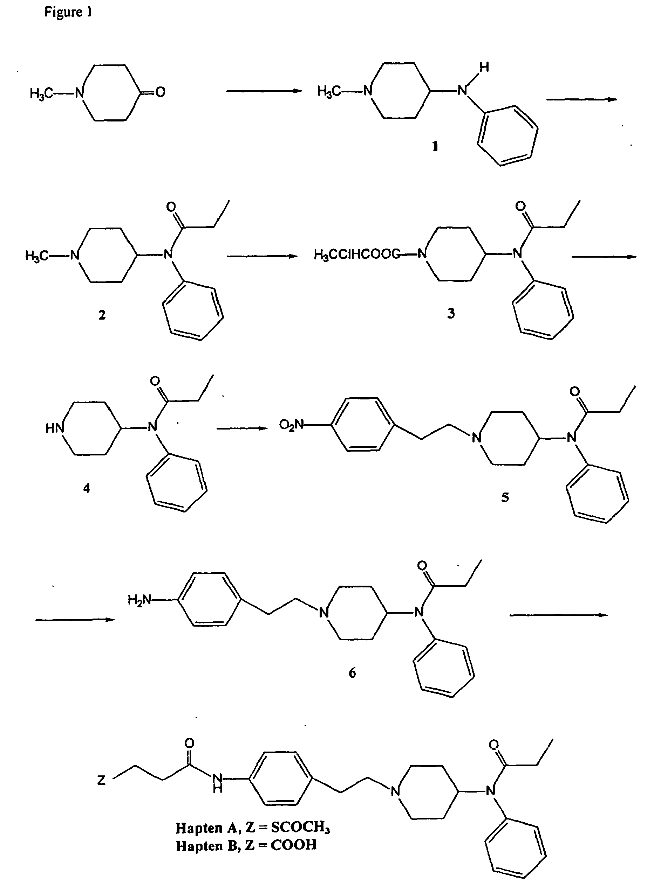

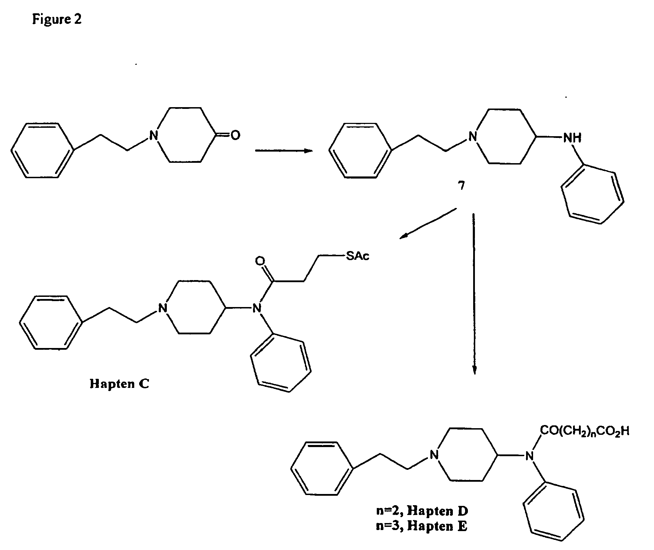

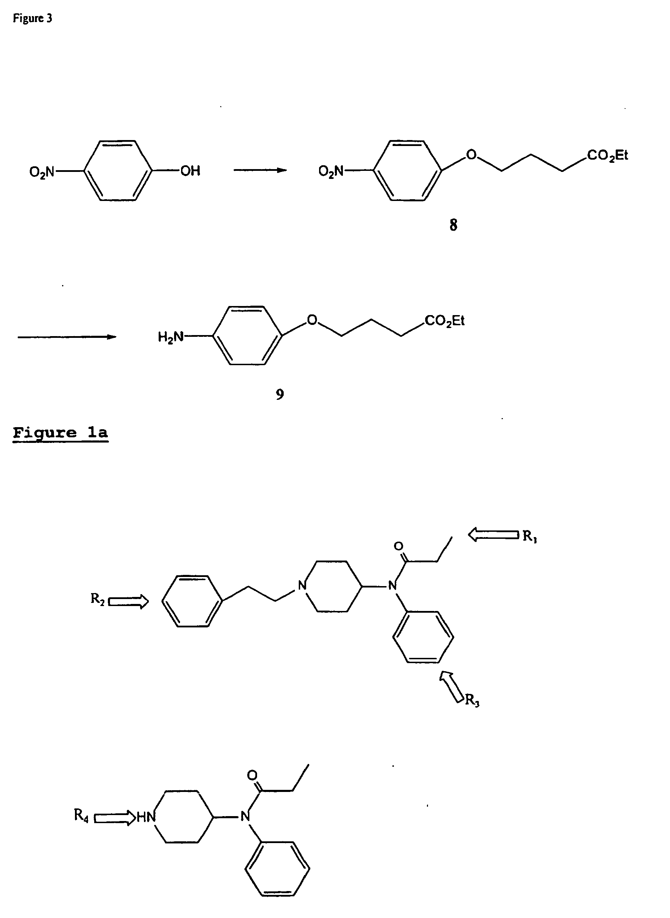

Position 1 is defined as the free or terminal end of the propionamide group of fentanyl. Position 2 is defined as the para position of the phenethyl group of fentanyl. Position 3 is defined as the para position of the N-phenyl group of fentanyl. Position 4 is defined as the piperidinyl-N of norfentanyl. Positions 1-4 are designated as R₁ - R₄, respectively in Figure 1a of the accompanying drawings.

Derivatisation positions 1, 2, 3 and 4 of the haptens described in the present invention are indicated below having regard to the structural formulae of fentanyl or nor-fentanyl:

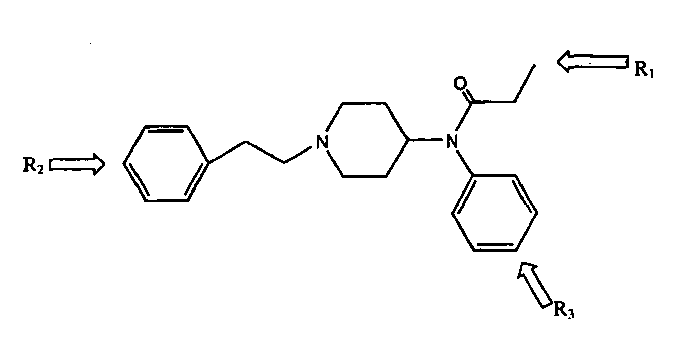

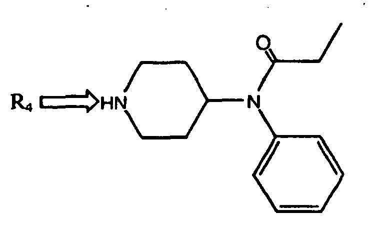

More specifically, in a first aspect, an immunogen is provided of the formula:- 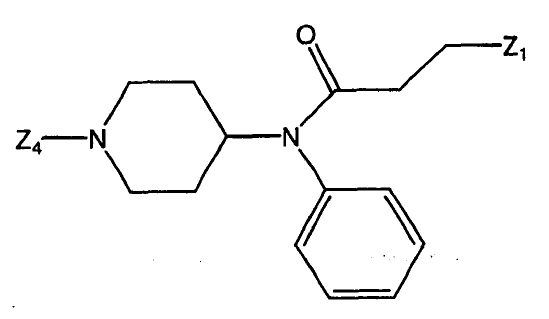 , in which Z₁ is a crosslinker coupled to an antigenicity-conferring carrier material or hydrogen; Z₄ is selected from N-phenethyl, a crosslinker coupled to an antigenicity-conferring carrier material or 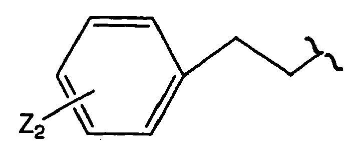 , in which Z₂ is a crosslinker coupled to an antigenicity-conferring carrier material at an ortho, a meta or apara position, with the provisos that, when Z₁ is hydrogen, Z₄ is a crosslinker coupled to an antigenicity-conferring carrier material or 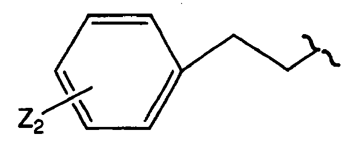 , in which Z₂ is a crosslinker coupled to an antigenicity-conferring carrier material at an ortho, a meta or a para position; and, when Z₄ is N-phenethyl, Z₁ is a crosslinker coupled to an antigenicity-conferring carrier material, the crosslinker not being -COOH. Preferably, the carrier material is a protein, a protein fragment, a synthetic polypeptide or a semi-synthetic polypeptide.

In a further aspect, a conjugate is provided of the formula:- 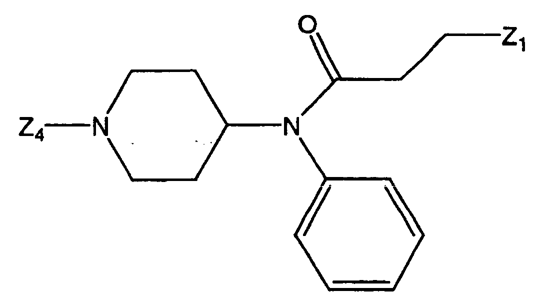 , in which Z₁ is a crosslinker covalently bonded to a detectable labelling agent, or hydrogen; Z₄ is selected from N-phenethyl, a crosslinker covalently bonded to a
detectable labelling agent or 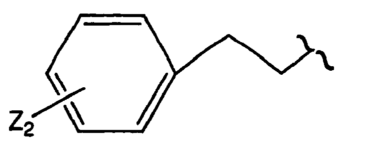 , in which Z₂ is a crosslinker covalently bonded to a detectable labelling agent at an ortho, a meta or a para position,
with the provisos that, when Z₁ is hydrogen, Z₄ is a crosslinker covalently bonded to a detectable labelling agent or 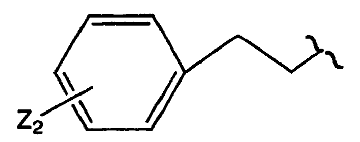 , in which Z₂ is a crosslinker covalently bonded to a detectable labelling agent at an ortho, a meta or a para position; and, when Z₄ is N-phenethyl, Z₁ is a crosslinker covalently bonded to a detectable labelling agent, the crosslinker not being -COOH. Preferably, the labelling agent is selected from an enzyme, a luminescent substance, a radioactive substance, or a mixture thereof. More preferably, the labelling agent is an enzyme, preferably a peroxidase, most preferably horseradish peroxidase. Alternatively, or additionally, the luminescent substance may be a bioluminescent, chemiluminescent or fluorescent material.

It will be appreciated that, in the haptens described, just one of the Z₁, Z₂ and Z₄ positions is a crosslinker and that, in immunogens and conjugates of the present invention, just one of the Z₁, Z₂ and Z₄ positions includes a crosslinker covalently linked to either an antigenicity-conferring carrier material or to a detectable labelling agent, respectively.

It will also be appreciated that, when Z₁ is hydrogen and Z₄ is N-phenethyl, the resultant compound is fentanyl and, when Z₁ and Z₄ are each hydrogen, the resultant compound is nor-fentanyl.

Table 1 hereunder summarises the crosslinkers and derivatisation positions for certain of the haptens described in the present invention and for certain of the immunogens and conjugates:

**Table 1: Chemical structures of certain haptens derived from fentanyl and from its metabolite, nor-fentanyl.**

| **DERIVATIVES** | **Z₁** | **Z₂** | **Z₄** |
|---|---|---|---|
| **HAPTEN A** | H | 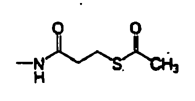 | 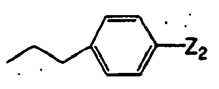 |
| **HAPTEN B** | H | 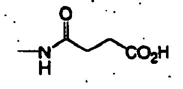 | 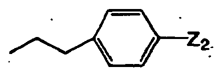 |
| **HAPTEN C** | 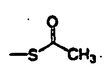 | H | 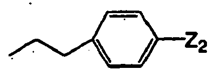 |
| **HAPTEN D** | -CO₂H | H | 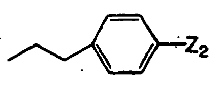 |
| **HAPTEN E** | 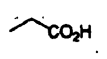 | H | 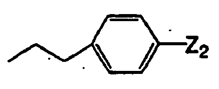 |
| **HAPTEN G** | H | | 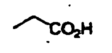 |

Preferably, the crosslinker of Z₂ is at the para position.

Preferably, the crosslinker of Z₁, Z₂ or Z₄ terminates, at its free end prior to coupling to the antigenicity-conferring carrier material or bonding to the labelling agent to form the immunogen or the conjugate, respectively, with -CO-R, in which R is hydroxyl or a short chain, straight or branched chain, alkyl moiety. By "short chain alkyl" is meant a C₁₋₅ moiety. More preferably, the short chain alkyl moiety is a straight chain moiety. Even more preferably, the short chain alkyl moiety is a C₁₋₂ moiety, preferably a C₁₋₂ straight chain alkyl moiety.

More preferably, the crosslinker of Z₁, Z₂ or Z₄ prior to coupling to the antigenicity-conferring carrier material or bonding to the labelling agent to form the immunogen or the conjugate, respectively, comprises:

-(NH)ₐ-(CO)_{b}-X_{c}-(CH₂)_{d}-Sₑ-CO-R

in which a is 0 or 1; b is 0 or 1; X is oxygen or sulfur; c is 0 or 1; d is selected from the integers 1-5; e is 0 or 1 and R is a short chain alkyl moiety, preferably a C₁₋₅ straight or branched chain alkyl moiety, or a hydroxy moiety. More preferably, the alkyl moiety is a C₁₋₅ straight chain moiety. Still more preferably, the alkyl moiety is a C₁₋₂ straight chain moiety. Most preferably, the alkyl moiety is methyl.

The last provisos, in their broadest aspect, for an immunogen or conjugate of the present invention, namely, that the Z₁ crosslinker is not carboxyl (-COOH) covalently linked to either an antigenicity-conferring carrier material or to a detectable labelling agent, respectively, means that the above-mentioned crosslinker formula excludes a crosslinker in which a, b, c, d and e are 0 and R is hydroxyl, when Z₁ is the crosslinker.

Preferably, when e is 1, c is 0.

Preferably a is 0; b is 0; and c is 0. More preferably, d is 0; e is 1 and R is methyl or, alternatively, d is 1; e is 0; and R is hydroxyl. More preferably, Z₁ or Z₄ is such a crosslinker. Alternatively, Z₂ could be such a crosslinker.

Alternatively, a is 1; b is 1; c is 0; and d is 2. Preferably, e is 1; and R is methyl or, alternatively, e is 0; and R is hydroxyl. More preferably, Z₂ is such a crosslinker at an ortho, a para or a meta position, preferably a para position. Alternatively, Z₁ or Z₄ could be such a crosslinker.

Further alternatively, a is 0; b is 0; e is 0; X is O; and c is 1. Preferably, d is 3; and R is hydroxyl. Z₁, Z₂ or Z₄ could be such a crosslinker.

The invention further provides a process of preparing the antibodies, the process comprising the steps of immunising an animal, preferably a vertebrate animal, most preferably a mammalian animal, by repeated administration of an immunogen according to the present invention, and collecting the resulting serum from the immunised animal. Preferably, the process further comprises fixing said serum antibodies to a backing substrate, preferably a solid support, most preferably a polystyrene solid support. Antibodies prepared in accordance with this process are polyclonal.

In a still further aspect, the present invention concerns antibodies raised against the immunogen of the present invention, the antibodies being capable of binding with at least one structural epitope of a metabolite of fentanyl or of a metabolite of its analogs. Preferably, the at least one structural epitope comprises the N-phenyl-N-(4-piperidinyl) amine structure of fentanyl. More preferably, the antibodies are fixed on a backing substrate.

The invention preferably concerns the production of avid polyclonal antisera to metabolites of fentanyl and to metabolites of its analogs, which antisera, most preferably, also exhibit some cross-reactivity with fentanyl and its analogs.

In a still further aspect, the present invention comprises a method for detecting, or determining the quantity of, metabolites of fentanyl, and metabolites of fentanyl analogs in a sample, the method comprising contacting the sample with the conjugate of the present invention, or a mixture thereof, and with antibodies of the present invention, or a mixture thereof; detecting, or determining the quantity of, bound conjugate; and deducing from a calibration curve the presence, or the amount of, metabolites of fentanyl and metabolites of fentanyl analogs in the sample.

In a further aspect, the invention includes a kit for detecting, or determining the quantity of, metabolites of fentanyl, and metabolites of fentanyl analogs, the kit including the conjugate of the present invention, or a mixture thereof and the antibodies of the present invention, or a mixture thereof. The kit may optionally include instructions for the use of said conjugates and said antibodies for detecting, or determining the quantity of, metabolites of fentanyl and metabolites of fentanyl analogs in a sample.

Preferably, the sample is a solution, such as a biological fluid. More preferably, the sample is serum or urine. Most preferably, the sample is a solution or a suspension from a human patient.

A method or kit may comprise a conjugate, or a mixture thereof, in which the or each conjugate is prepared from a hapten in which one of Z₁, Z₂ or Z_{4,} preferably one of Z₂ or Z₄, is a crosslinker and an antibody, or a mixture thereof, in which the or each antibody is generated to a hapten, in which one of Z₁, Z₂ or Z_{4,} preferably one of Z₁ or Z₂, is a crosslinker. The respective constituent haptens may be identical or the respective constituent haptens may be derivatised at the same position but using different crosslinkers, the latter being preferred. However, it is preferred that the respective constituent haptens be derivatised at different positions, either using the same crosslinkers or using different crosslinkers. More preferred, is an immunogen derived from a hapten in which Z₂ is a crosslinker for use in a method or kit with a conjugate derived from a hapten in which one of Z₂ or Z₄ is a crosslinker, in which the respective crosslinkers (of the immunogen and the conjugate) are the same or, more preferably, different. Preferred, is an immunogen derived from a hapten in which Z₂ is a crosslinker for use in a method or kit with the conjugate derived from a hapten in which Z₄ is a crosslinker, in which the respective crosslinkers (of the immunogen and the conjugate) are the same or, more preferably, different.

Equally contemplated, is a conjugate derived from a hapten in which Z₂ or Z₄ is a crosslinker for use in a method or kit of the present invention with an immunogen derived from a hapten in which one of Z₁, Z₂ or Z₄ is a crosslinker, in which the respective crosslinkers (of the immunogen and the conjugate) are the same or, more preferably, different. Still more preferred, is a conjugate derived from a hapten in which Z₂ or Z₄ is a crosslinker for use in a method or kit according to the present invention, with an immunogen derived from a hapten in which one of Z₁ or Z₂ is a crosslinker. Most preferred, is a conjugate derived from a hapten in which Z₂ or Z₄ is a crosslinker for use in a method or kit of the present invention with an immunogen derived from a hapten in which Z₂ is a crosslinker, in which the respective crosslinkers (of the immunogen and the conjugate) are the same, or, more preferably, different.

In a further aspect, the present invention involves use of the conjugates according to the present invention, or a mixture thereof, with the antibodies according to the present invention, or a mixture thereof, to test samples such as biological fluids, for detecting, or determining the quantity of, metabolites of fentanyl and metabolites of fentanyl analogs.

The present invention relates to novel haptens, which are employed in the preparation of novel immunogens by conjugation to conventional antigenicity-conferring carrier materials. The resulting immunogen is then administered to animals, preferably vertebrate hosts, most preferably mammalian hosts, to elicit production of avid polyclonal antisera which are then used to develop a generic immunoassay for metabolites of fentanyl and for metabolites of fentanyl analogs, employing a conjugate (hapten - detectable labelling agent) as the detection reagent.

The focus is the preparation of antibodies specific for metabolites of fentanyl and for metabolites of fentanyl analogs. In order to achieve such broad specificity, haptens and immunogens are generated by modification of fentanyl using Z₁ or Z₂ as the crosslinker, and by modification of norfentanyl using Z₄ as the crosslinker.

### Preparation of Haptens

Preparation of haptens A and B is performed according to reaction scheme 1 set out in Figure 1. Preparation of haptens C, D and E is performed according to reaction scheme 2 set out in Figure 2. Preparation of hapten F and immunogen F is performed according to reaction schemes 3 and 4 set out in Figures 3 and 4 and preparation of hapten G and immunogen G is performed according to reaction scheme 5 set out in Figure 5.

Referring to Figure 1, haptens N-[1-(*p*-acetylthiopropionamido)phenethyl-4-piperidinyl]-N-phenylpropionamide (A) and N-[1-(*p*-succinamido)phenethyl-4-piperidinyl]-N-phenylpropionamide (B) are prepared according to reaction scheme-1. A condensation reaction between aniline and N-methyl-4-piperidone, via Schiff base formation, in the presence of 1,2-dichloroethane, acetic acid and NaBH(OAc)₃ results in the production of compound 1. This compound reacts with propionic anhydride, in the presence of toluene and under reflux to produce compound 2. Nor-fentanyl (compound 4) is prepared by N-dismethylation of compound 2, in a two step process, using in the first step, 1-chloroethylchloroformate (ClCO₂CHClCH₃) in the presence of 1,2-dichloroethane, under reflux for 2 hours and, in the second step, methanol. Reaction of compound 4 with *p*-nitrophenethylbromide in the presence of 4-methyl-2-pentanone and K₂CO₃ under reflux; followed by reduction of the nitro group to an amino group using Pd-C in the presence of HCOONH₄ and CH₃OH results in production of intermediate compound 6. Hapten A is produced by reaction of compound 6 with N-succinimidyl 3-(acetylthio)propionate (SATP) in the presence of EDPA and dioxane and hapten B is produced by reaction of compound 6 with succinic anhydride.

Referring to Figure 2, the haptens N-(1-phenethyl-4-piperidinyl)-N-phenyl(S-acetylthiopropionamide) (C), N-(1-phenethyl-4-piperidinyl)-N-phenylsuccinamide (D) and N-(1-phenethyl-4-piperidinyl)-N-phenylglutaramide (E) are prepared according to reaction scheme-2. Despropionyl fentanyl (compound 7) is prepared by reaction of 1-phenethyl-4-piperidone with aniline followed by reduction of the Schiff base. The compound produced is then reacted with N-succinimidyl 3-(acetylthio)propionate (SATP) in the presence of EDPA and dioxane to yield hapten C, with succinic anhydride to yield hapten D or with glutaric anhydride to yield hapten E.

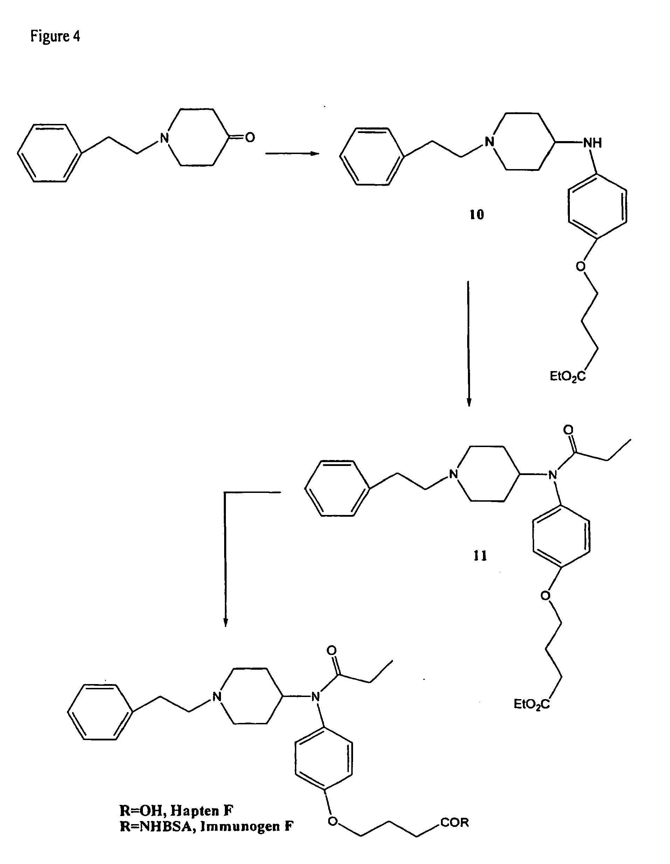

Referring to Figures 3 and 4, prior to the preparation of the hapten N-(1-phenethyl-4-piperidinyl)-N-(*p*-O-carboxypropyl)-phenylpropionamide (F), [ethyl-*p*-(O-carboxypropyl)] aniline (compound 9) is generated according to reaction scheme-3 of Figure 3. *p*-nitrophenol is alkylated with ethyl-4-bromobutyrate in the presence of sodium hydride to produce compound 8. The nitro group of compound 8 is then reduced to an amino group using Pd-C, resulting in the production of compound 9.

N-(1-phenethyl-4-piperidinyl)-N-(*p*-O-carboxypropyl)-phenylpropionamide (hapten F) is prepared in three steps according to reaction scheme-4 of Figure 4.

N-(1-phenethyl)-4-piperidone is reacted with [ethyl-*p*-(O-carboxypropyl)]aniline (compound 9) in 1,2-dichloroethane, in the presence of sodium triacetoxyborohydride and acetic acid, to produce compound 10. Compound 10 reacts with propionic anhydride in toluene under reflux to yield compound 11. Hapten F is produced by saponification of compound 11 with 2N sodium hydroxide in methanol.

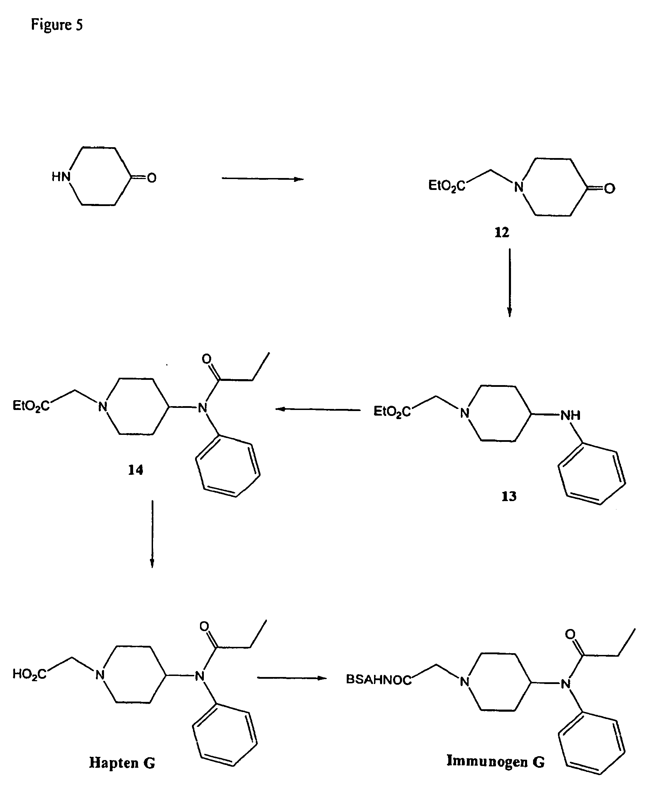

Referring to Figure 5, the hapten N-(1-carboxymethyl-4-piperidinyl) N-phenylpropionamide (G) is prepared in four steps according to reaction scheme-5. Reaction of 4-piperidone monohydrate monohydrochloride with ethyl bromoacetate in the presence of sodium hydride in DMF yields ethyl-N-carboxymethyl-4-piperidone (compound 12). Compound 12 is then reacted with aniline in the presence of sodium triacetoxyborohydride and acetic acid to produce N-(ethyl-1-carboxymethyl-4-piperidinyl)-N-phenylalanine (compound 13). Compound 13 is treated with propionic anhydride in toluene under reflux to yield compound 14. Hapten G is produced by saponification of compound 14 with sodium hydroxide (2N) in methanol.

### Preparation of Immunogens and Conjugates

Although the fentanyl haptens provide defined structural epitopes, they are not in themselves immunogenic and therefore need to be conjugated to carrier material, which will elicit an immunogenic response when injected into a host animal. Suitable carrier materials include proteins such as albumins, serum proteins e.g. globulins, ocular lens proteins and lipoproteins. Illustrative protein carriers include bovine serum albumin, egg ovalbumin, bovine gamma globulin, thyroxine binding globulin, keyhole limpet haemocyanin etc. Alternatively, synthetic poly(amino acids) having a sufficient number of available amine groups such as lysine may be employed, as may other synthetic or natural polymeric materials bearing reactive functional groups. In particular, carbohydrates, yeasts or polysaccharides may be conjugated to the hapten to produce an immunogen.

Each hapten can also be covalently linked to a labelling group such as an enzyme (for example, horse radish peroxidase), a substance having fluorescent properties or a radioactive label to produce conjugates (also known as detection reagents) for use in the immunoassays. The fluorescent substance may be, for example, a monovalent residue of fluorescein or a derivative thereof.

In order to confirm that adequate conjugation of hapten to carrier material has been achieved, prior to immunisation, each immunogen is evaluated using matrix-assisted UV laser desorption/ionization time-of-flight mass spectrometry (MALDI-TOF MS). In the case of the preferred carrier material, bovine serum albumin, a minimum of 6 molecules of hapten per carrier molecule is preferred.

In preparing conjugates or immunogens with haptens where e, in the crosslinker, is 1 such as, for example, haptens A and C, maleimide, halo, mercaptopyridyl, or vinylsulphone groups must first be introduced to the labelling agent or carrier material, respectively, using heterobifunctional linkers such as, but not limited to: N-(γ-maleimidobutyryloxy)succinimide ester (GMBS); succinimidyl 4-(N-maleimidomethyl)cyclohexane-1-carboxylate (SMCC); (m-maleimidobenzoyl)-N-hydroxysuccinimide (MBS); succinimidyl 4-(*p*-maleimidophenyl)butyrate (SMPB); N-succinimidyl(4-iodoacetyl)aminobenzoate (SIAB); bromoacetylglycine N-hydroxysuccinimide; N-succinimidyl 3-(2-pyridyldithio)propionate (SPDP); vinylsulphone (Pierce Chemical Company, USA). The thus-modified labelling agent or carrier material, can then be conjugated via the thiol groups on haptens in which e is 1, such as haptens A and C. For haptens where e, in the crosslinker, is 0, such as haptens B, D, E, F and G, conjugation is performed without prior-modification of labelling agent or carrier material, as appropriate, using standard methods of conjugation such as EDC or mixed anhydride.

### Preparation of Antisera

In order to generate polyclonal antisera, the immunogen is mixed with Freund's Adjuvant and the mixture is injected into a host animal, such as a rabbit, sheep, mouse, guinea pig or horse. Further injections (boosts) are made and serum is sampled for evaluation of antibody titer. When the optimal titer has been reached, the host animal is then bled to yield a suitable volume of specific antiserum. The degree of antibody purification required depends on the intended application. For many purposes, there is no requirement at all for purification, however, in other cases, such as where the antibody is to be immobilized on a solid support, purification steps can be taken to remove undesired material and eliminate non specific binding.

The specific antibodies of the present invention are useful as reagents in biochemical assays for the detection, or for the determination of the amount of, metabolites of fentanyl and metabolites of its analogs in biological fluids.

In the following table, the results of characterising data for compounds prepared in the following Examples 4, 6-11 and 13-15 are set out:

| **Compound** | Melting Point (°C) | FT IR (KBr, Diffuse Reflectance, cm⁻¹) |
|---|---|---|
| Example 6 | 154-157 | 3479.64, 3360.85, 1648.53 and 1594.23 |
| Example 7 (Hapten A) | 164-167 | 3322.61, 1687.21, 1627.29 and 1594.98 |
| Example 4 | 94-96 | 3426.96, 1648.67 and 1594.62 |
| Example 9 | 92-94 | 3286.45, 1601.08 |
| | | |
| Example 8 (Hapten B) | 222-225 | 3244.1, 3180.1, 1677.7,1648.7 and 1597.1 |
| Example 10 (Hapten C) | | 1687.02, 1627.42 and 1536.36 |
| Example 11 (Hapten D) | 198-202 | 1714.06,1650.94 and 1595 |
| Example 11 (Hapten E) | 141-142 | 3442.02, 1654.13 and 1596.14 |
| Example 13 | 198-200 | 3330.98, 1735.92, 1614.7 and 1514.78 |
| Example 14 | 183-185 | 1730.99, 1654.98 and 1509.46 |
| Example 15 (Hapten F) | 113 (Decomp) | 3228, 1716,1, 1647.9 and 1510 |

### General Procedure for MALDI-TOF Analysis of Immunogens

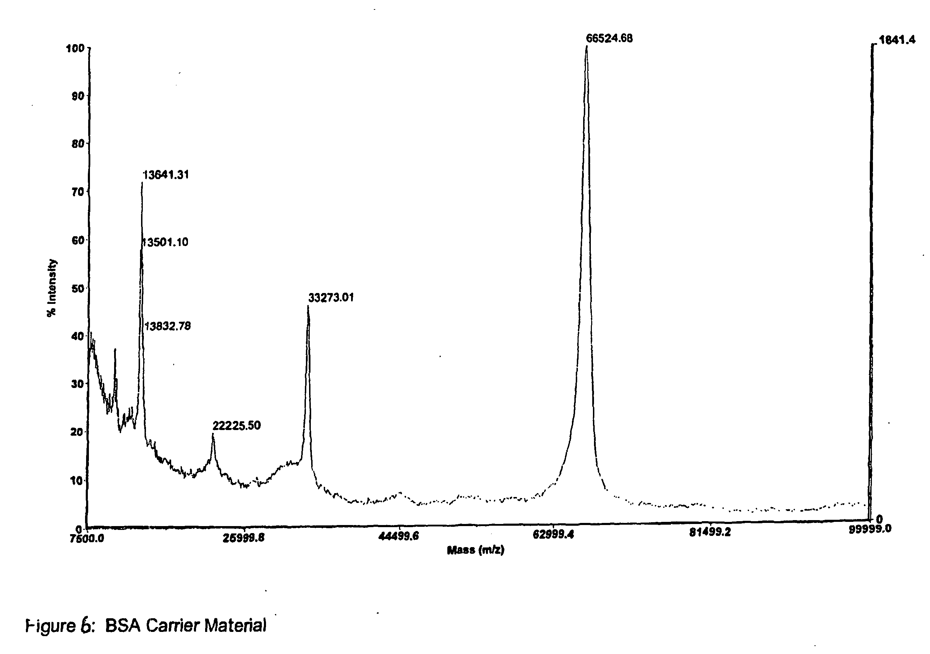

MALDI-TOF mass spectrometry was performed using a Voyager STR Biospectrometry Research Station laser-desorption mass spectrometer coupled with delayed extraction. An aliquot of each sample to be analysed was diluted in 0.1% aqueous trifluoroacetic acid (TFA) to create 1mg/ml sample solutions. Aliquots (1µl) were analysed using a matrix of Sinapinic acid and bovine serum albumin (Fluka) was used as an external calibrant. Figure 6 of the accompanying drawings shows the analysis for BSA carrier material. As will be seen, a major signal was present which indicates an average protonated mass for this sample of m/z 66,525. The signal at m/z 33,273 is consistent with the major component in a doubly-charged form and further signals were observed including that at m/z 13,641.

In the following examples, all percentages are v/v unless otherwise specified.

### EXAMPLE 1: Reaction Scheme 1

### Preparation of N-(1-methyl)-4-(phenylamine)piperidine (Compound 1)

To a solution of 4-N-methyl-1-piperidone (7g, 0.0062mol) in 100ml of anhydrous 1,2-dichloroethane, was added aniline (12.7g, 0.14mol) and acetic acid (4ml), followed by sodium triacetoxyborohydride (13.1g, 0.062mol). The mixture was stirred at room temperature under nitrogen overnight.

After evaporation of the solvent under reduced pressure, water (100ml) was added and the solution was made alkaline by adding NaOH (1N), to pH 9. The solution was then extracted using toluene (2x 100ml), the organic layers combined and washed with water (150ml), brine (50ml), dried over sodium sulfate, filtered and concentrated under reduced pressure. The residue was chromatographed (silica gel, 5%MeOH/ chloroform) to give 9.4g (80% yield) of compound 1 as a white solid after recrystallization from ethyl acetate.

### EXAMPLE 2: Reaction Scheme 1

### Preparation of N-(1-methyl-4-piperidinyl)-N-phenylpropionamide (Compound 2)

To a solution of compound 1 of Example 1 (9.0g, 0.047mol) in anhydrous toluene (150ml), was added propionic anhydride (15.4g, 0.118mol). The mixture was stirred overnight at reflux. The solvents were removed *in vacuo* and the residue recrystallized from ethyl acetate/hexane to give 10g of compound 2 as a white solid (86% yield).

### EXAMPLE 3: Reaction Scheme 1

### Preparation of N-[1-(1'-ethylchloroformate)-4-piperidinyl]-N-phenylpropionamide (Compound 3)

10g (0.04mol) of compound 2 of Example 2 was dissolved in anhydrous 1,2-dichloroethane (100ml), under a nitrogen atmosphere, and cooled to 0°C in an icebath. To this solution was added, dropwise, 1-chloroethyl chloroformate (6.6g, 0.046mol) and the solution heated at reflux for 2 hours (thin layer chromatography indicated when reaction was complete). The solution was cooled to room temperature and the solvents removed under reduced pressure. The viscous oily residue was purified by column chromatography (silica gel, 10% ethyl acetate/90% n-hexane) to give the title compound as a white amorphous solid (10g, 74% yield).

### EXAMPLE 4: Reaction Scheme 1

### Preparation of norfentanyl (Compound 4)

10g (0.03mol) of compound 3 of Example 3 was dissolved in anhydrous methanol (150ml) and stirred overnight at room temperature. The solution was concentrated under reduced pressure to give the title compound as a white powder in quantitative yield.

### EXAMPLE 5: Reaction Scheme 1

### Preparation of N-[1-(p-nitrophenethyl)-4-piperidinyl]-N-phenylpropionamide (Compound 5)

A suspension of norfentanyl of Example 4 (6.71g, 0.029mol), *p-*nitrophenethylbromide (7.178g, 0.0372mol), potassium carbonate (19g, 0.14mol) and a few crystals of sodium iodide in 4-methyl-2-pentanone (200ml) was stirred overnight at reflux, under nitrogen. After cooling to room temperature, the precipitate was removed by filtration and the solution concentrated under reduced pressure. The dark residue was dissolved in ethyl acetate (200ml) and washed with water (2 x 100ml), dried over sodium sulfate, filtered and the solvent removed *in vacuo*. The residue was purified by column chromatography (silica gel, 10%MeOH/90%CHCl₃ v/v) to give the title compound as a yellow solid (5.33g, 56% yield).

### EXAMPLE 6: Reaction Scheme 1

### Preparation of N-[1-(p-aminophenethyl)-4-piperidiny]-N-phenylpropionamide (Compound 6)

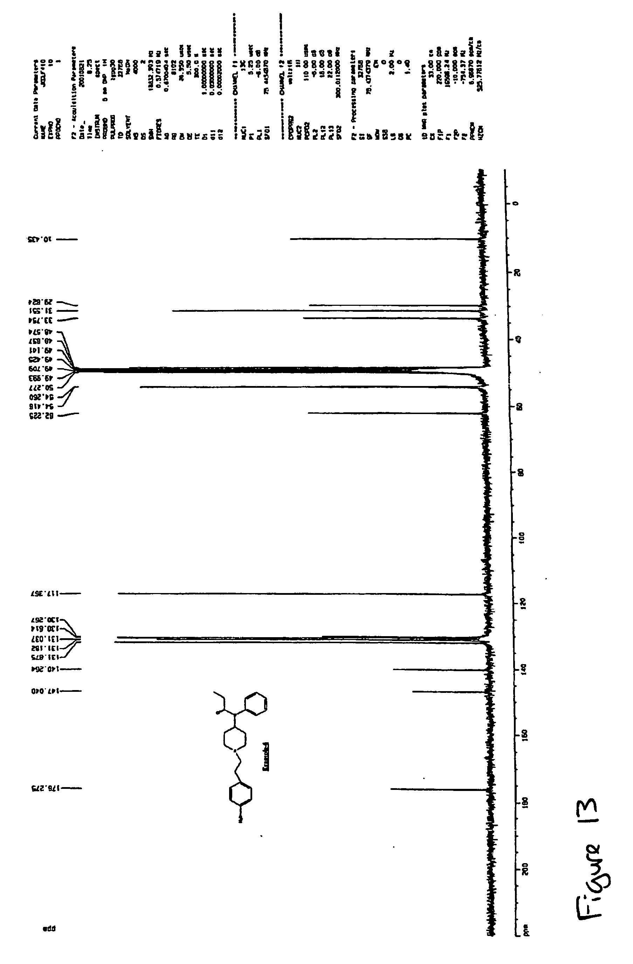

To a stirring solution of compound 5 of Example 5 (5g, 0.013mol) in anhydrous methanol (200ml) was added, under a nitrogen atmosphere, 10%Pd-C (650mg) followed by ammonium formate (5g, 0.063mol). The mixture was stirred at room temperature for 4 hours and filtered through a short column of Celite^{™} to remove the catalyst. The solvent was removed under reduced pressure and the residue taken up in water (100ml). The aqueous solution was made alkaline by the addition of 1N NaOH and extracted with ethyl acetate (2 x 100ml). The organic layers were combined, washed with water (50ml), brine (50ml) and dried over anhydrous sodium sulfate. The solution was then concentrated under reduced pressure to produce compound 6, a white powder exhibiting a single spot on thin layer chromatography (2.7g, 60% yield).
NMR data for compound 6 are presented in Figure 13.

### EXAMPLE 7: Reaction Scheme 1

### Preparation of N-[1-(p-acetylthiopropionamido)phenethyl-4-piperidinyl]-N-phenylpropionamide (Hapten A)

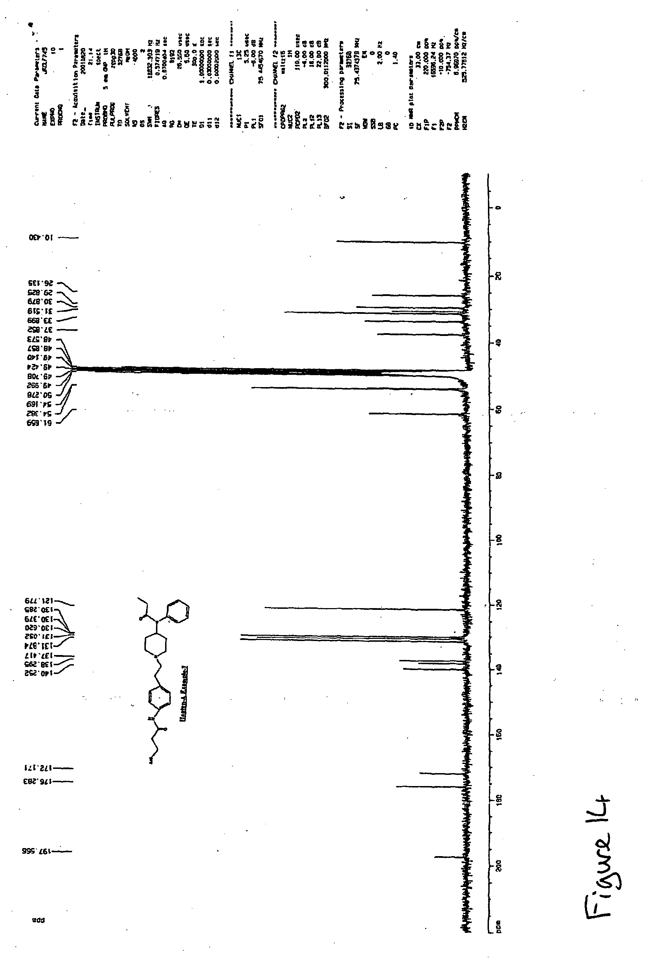

To a stirring solution of compound 6 of Example 6 (1g, 0.0028mol) and N-succinimidyl 3-(acetylthio)propionate (SATP) (0.824g, 0.00336mol) in 25ml of anhydrous dioxane under nitrogen was added diisopropylethylamine (EDPA) (0.72ml, 0.0042mol), and the mixture was stirred and heated at 60°C for 6hrs. The mixture was then concentrated *in vacuo* and the residual was purified by flash chromatography on silica gel using 10% v/v methanol in chloroform to produce hapten A, a white solid which was recrystallized from hexane chloroform in the cold (835mg, 62% yield).
NMR Data for Hapten A are presented in Figure 14.

### EXAMPLE 8: Reaction Scheme 1

### Preparation of N-[1-(p-succinamido)phenethyl-4-piperidinyl]-N-phenylpropionamide (Hapten B)

To a solution of compound 6 of Example 6 (1g, 0.0028mol) in anhydrous benzene (75ml) was added succinic anhydride (0.7007g, 0.007mol) and the mixture was refluxed overnightA white precipitate was produced and TLC confirmed that all starting material had been consumed. The precipitate was filtered off, washed with benzene and dried. It was taken up in water (20ml) and heated at 50°C for one hour, filtered and thoroughly dried. The solid was taken up in acetonitrile (20ml) and heated at 60°C for one hour, filtered, washed with a little cold acetonitrile and dried under vacuum overnight to give the title compound as a white solid (0.82g, 60% yield).

### EXAMPLE 9: Reaction Scheme 2

### Preparation of N-(1-phenethyl-4-piperidinyl)-phenylamine (despropionylfentanyl) (Compound 7)

The title compound was prepared by the method outlined in Example 1 using 1-phenethyl-4-piperidone (5.3g, 0.026mol), aniline (5.01ml, 0.55mol), acetic acid (3ml) 1, 2-dichloroethane (100ml) and sodium triacetoxyborohydride (5.82g, 0.027mol). The title compound was obtained as a white solid (74% yield).

### EXAMPLE 10: Reaction Scheme 2

### Preparation of N-(1-phenethyl-4-piperidinyl)-N-phenyl-(S-acetylthiopropionamide) (Hapten C)

Hapten C was prepared by the same method outlined in Example 7 with 55% yield, i.e., with N-succinimidyl 3-(acetylthio) propionate (SATP) in anhydrous dioxane, to which diisopropylethylamine (EDPA) is added.

### EXAMPLE 11: Reaction Scheme 2

### Preparation of Haptens D and E

Haptens D and E were prepared by the same method outlined in Example 8. Hapten D was prepared using succinic anhydride (2eq) and Hapten E prepared using glutaric anhydride (2eq).

### EXAMPLE 12: Reaction Scheme 3

### Preparation of [Ethyl-p-(O-carboxypropyl)]aniline (Compound 9)

To a suspension of sodium hydride (3.696g, 0.11mol) in 100ml of anhydrous dimethylformamide under nitrogen, was added dropwise *p*-nitrophenol (13.911g, 0.1mol) in 100ml of DMF. The mixture was heated at 60°C for 1 h (no evolution of hydrogen) and then cooled to room temperature. To this mixture was added dropwise ethyl-4-bromobutyrate (23.4g, 0.12mols) in 50ml of DMF over a period of 15 mins. The mixture was again heated at 60 °C and stirred for 4 hrs. After cooling to room temperature, the solvents were evaporated under reduced pressure. 150 ml water was added to the crude product which was then extracted using ethyl acetate (2x 150ml). The combined organic layers were washed with brine, dried and filtered. The solvent was removed *in vacuo* and the residue purified by flash chromatography (90% hexane / 10% ethyl acetate v/v) to give 21.7g (86% yield) of [ethyl-*p*-(O-carboxypropyl)-1-nitrophenyl] (compound 8) as a white solid.

To a stirring solution of compound 8 (9.87g, 0.039 mol) in 400ml of anhydrous methanol was added, under nitrogen, Pd-C (10%)(1.95g) followed by ammonium formate (15g, 0.189mols). The mixture was stirred at room temperature for 2 hours, after which TLC confirmed that all starting materials had been consumed. The catalyst was removed by filtration over Celite^{™}. The solvent was then removed under vacuum and the residue taken up in water (150ml). The aqueous solution was made alkaline by the addition of 2N sodium hydroxide and extracted by ether (2x150ml). The organic layers were combined, washed with water (100ml), brine (100ml) and dried over anhydrous sodium sulfate. The solution was then concentrated under reduced pressure to give a white solid of compound 9 (6.52g, 75% yield).

### EXAMPLE 13: Reaction Scheme 4

### Preparation of N-[(1-phenethyl-4-piperidinyl)-N-(ethyl p-(O-carboxypropyl))]phenylamine (Compound 10)

Compound 10 was prepared in the same manner as outlined in Example 1 using compound 9 instead of aniline and 1-phenethyl-4-piperidone, in the presence of anhydrous 1,2-dichloroethane, acetic acid and sodium triacetoxyborohydride. Compound 10 was obtained in crystal form (65% yield).

### EXAMPLE 14: Reaction Scheme 4

### Preparation of [ethyl p-(O-carboxypropyl)]fentanyl (Compound 11)

The title compound was prepared in the same manner as outlined in Example 2, using compound 10 in anhydrous toluene and propionic anhydride under reflux. The title compound 11 was obtained as a white solid in 80% yield.

### EXAMPLE 15: Reaction Scheme 4

### Preparation of p-[(O-carboxypropyl)]fentanyl (Hapten F)

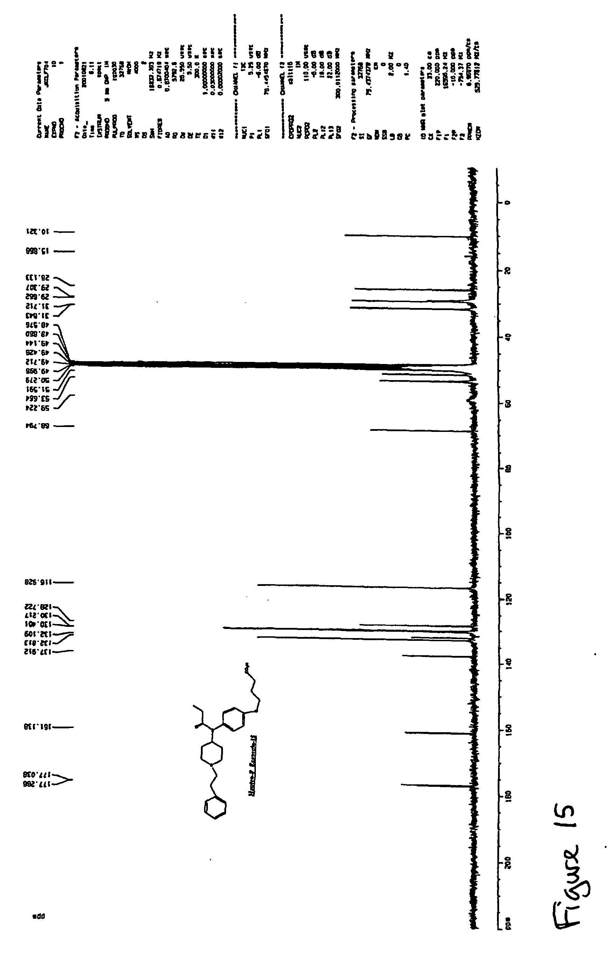

To a solution of compound 11 (3.5g, 7.5mmol) in methanol (80ml) was added 2N sodium hydroxide (20ml) and the mixture stirred at room temperature for 4 hours (TLC showed no starting materials were left). The mixture was reduced to dryness *in vacuo*, water (50ml) was added and the pH of the resulting solution adjusted to 6. The solution was extracted with chloroform (2 x 100ml), the combined organic extracts were washed with brine (1 x 50ml), dried over sodium sulfate, filtered and concentrated *in vacuo*. The solid obtained was triturated with ether, filtered and dried overnight to give 2.1g (64% yield) of hapten F.
NMR Data for hapten F are presented in Figure 15.

### EXAMPLE 16: Reaction Scheme 5

### Preparation of ethyl (1-carboxymethyl)-4-piperidone (Compound 12)

To a solution of 4-piperidone monohydrate monohydrochloride (12g, 78.12mmol) in anhydrous dimethylformamide (120ml) under nitrogen was added, in small portions, sodium hydride [60% w/w dispersion in mineral oil] (5.25g, 156mmol). After complete addition, the mixture was heated at 60°C for 1 hour (no further hydrogen gas was seen to be evolved), cooled to room temperature and ethyl bromoacetate (19.6g, 177mmol) in DMF (50ml) added dropwise over 15 minutes. The resulting mixture was heated at 60°C overnight. A few drops of water were added to quench the reaction and the solvents were removed *in vacuo*. Water (100ml) was added and the mixture extracted with ethyl acetate (2 x 100ml). The combined organic layers were washed with water (1 x 100ml), brine (1 x 100ml), dried over sodium sulfate and the solvent removed *in vacuo*. The residue was purified by column chromatography (50% ethyl acetate / 50% hexane v/v) to give the title compound 12 as a clear oil (3.4g, 65% yield)

### EXAMPLE 17: Reaction Scheme 5

### Preparation of N-[ethyl-1-carboxymethyl)-4-(phenylamino)]piperidine (Compound 13)

The compound 13 was prepared in the same manner as outlined in Example 1, using ethyl (1-carboxymethyl)-4-piperidone (compound 12) (7.4g, 0.04mol) and aniline (8.2g, 0.14mol) in 1,2-dichloroethane (120ml) in the presence of sodium triacetoxyborohydride (8.5g, 0.04mol) and acetic acid (4ml). The title compound was obtained after purification by column chromatography (silica gel, 10% v/v methanol in chloroform) (8.4g, 80% yield).

### EXAMPLE 18: Reaction Scheme 5

### Preparation of N-(ethyl-carboxymethyl)-norfentanyl (Compound 14)

Compound 14 was prepared from compound 13 (7.86g, 0.03mol) in anhydrous toluene and propionic anhydride (8.59g, 0.066mol) using the same method as given in Example 2. Compound 14 was obtained after recrystallisation from ethyl acetate / hexane (7.15g, 75% yield).

### EXAMPLE 19: Reaction Scheme 5

### Preparation of N-(carboxymethyl)-norfentanyl (Hapten G)

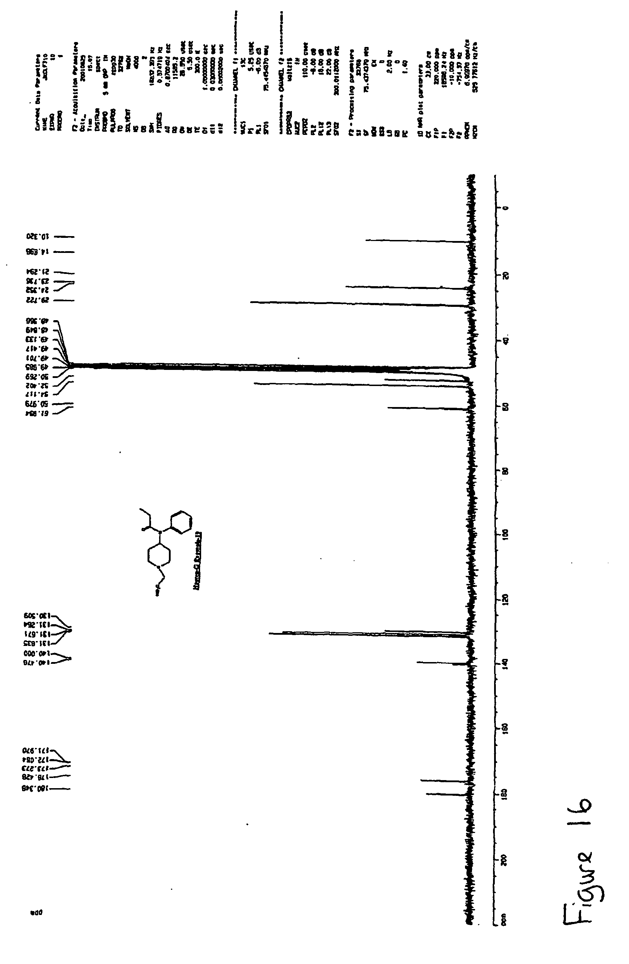

To a solution of compound 14 (3.78g, 10mmol) in methanol (80ml) was added 2N sodium hydroxide (20ml) and the mixture stirred at room temperature for 4 hours (TLC showed no starting materials present). The mixture was reduced to dryness, water (50ml) was added and the pH adjusted to 5 by addition of 1N HCl. The precipitate which formed was collected by filtration, washed with a little cold water and dried overnight in the presence of phosphorous pentoxide. The white solid obtained corresponds to hapten G (1.38g, 50% yield).
NMR data for hapten G are presented in Figure 16.

### EXAMPLE 20

### Preparation of bovine serum albumin (BSA) - bromoacetylglycine

To a solution of BSA (1g) in 0.1M borate buffer (pH 8.5, 45ml), cooled to 0°C, was added dropwise N-succinimidyl bromoacetylglycine (0.375g, 0.13mmol) in DMF (5ml). During the addition the pH was maintained at 8. After complete addition, the pH was stabilized at 8 and the solution was stirred at 0°C for one hour. The pH was then adjusted to approximately 7 and the solution dialysed overnight at 4°C against distilled water (2 changes). The solution was then freeze dried to give approximately 1g of BSA modified by bromoacetylglycine.

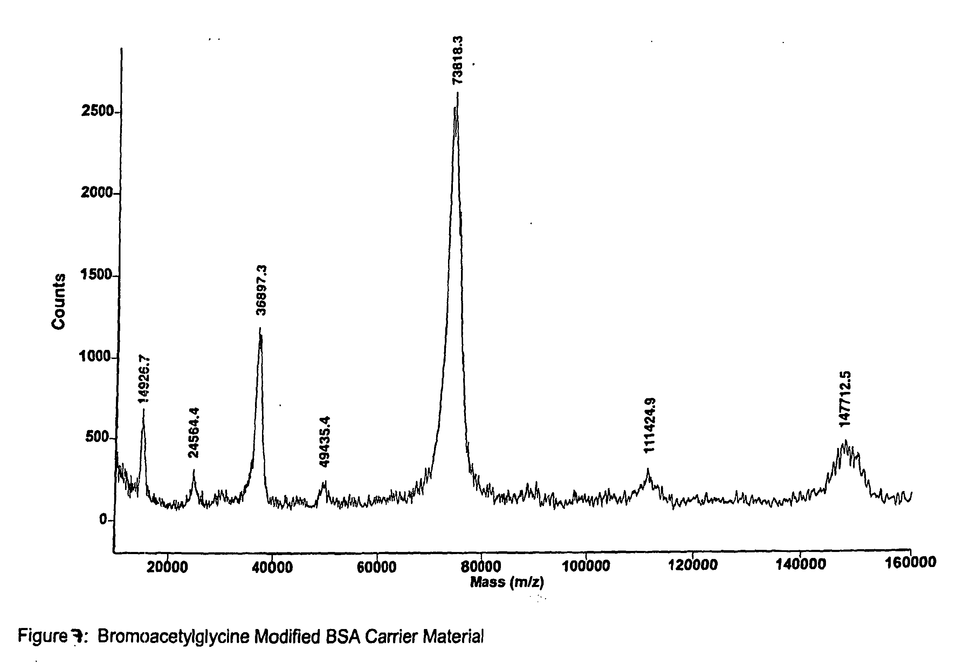

MALDI-TOF analysis (see Figure 7) shows that a major signal was present which indicates an average protonated mass for this sample of m/z 73,818. The signals at m/z 24,564, 36,897 and 147,713 are consistent with the major component in triply-charged, doubly-charged and dimer forms respectively. Further signals were observed including those at m/z 14,927, 49,425 and 111,425.

These data suggest that 40.3 lysine groups per molecule of BSA have been modified by the bromoacetylglycine.

### EXAMPLE 21

### Production of immunogen A using bromoacetylglycine modified BSA

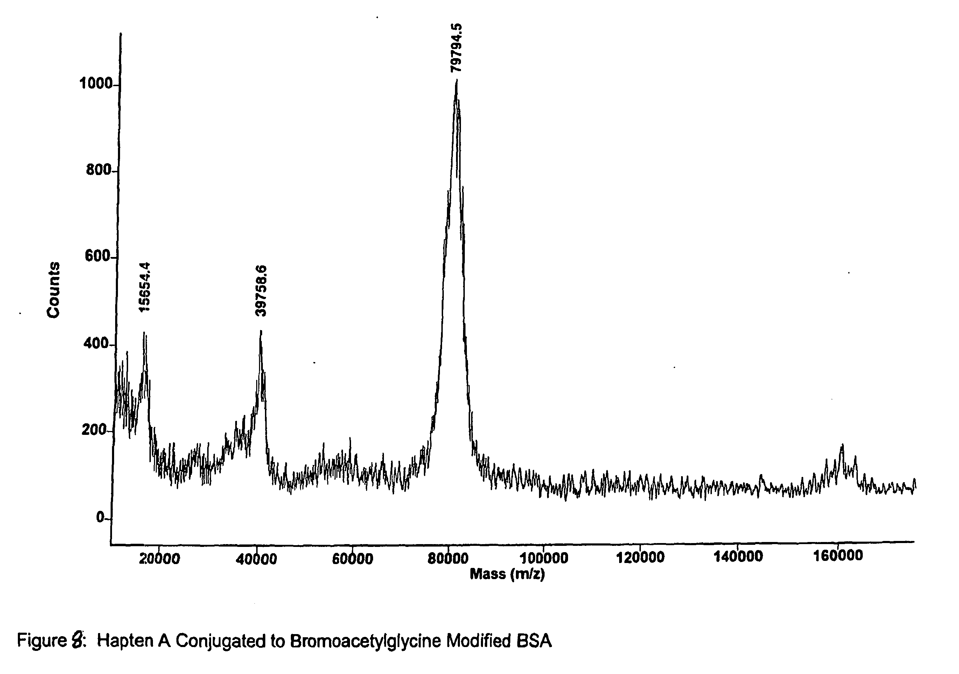

Hapten A (58.15mg, 0.12mmol) was dissolved in anhydrous DMF (100µL) and to this solution was added hydroxylamine solution (900µL, pH 12). The mixture was allowed to stand 10-15 mins (TLC showed disappearance of Hapten A and the formation of a new compound of lower Rf). Phosphate buffer was added to quench the reaction and the pH adjusted to 7 by the addition of 0.5M HCl. This solution was added dropwise to a solution of the modified BSA of Example 20 (200mg in 10ml of water) and the solution stirred at 4°C overnight (protected from light). The solution was then dialysed against distilled water for 24 hours (3 changes) and freeze-dried. MALDI results (see Figure 8 of the accompanying drawings) showed 12.5 molecules of Hapten A had been conjugated to one molecule of modified BSA. Specifically, a signal was present which indicates an average protonated mass for this sample of m/z 79,795. The signal at m/s 39,759 is consistent with the major component in doubly-charged form. A further signal was observed at m/z 15,654.

### EXAMPLE 22

### Production of Immunogen C using Bromoacetylglycine modified BSA

The conjugation was carried out by the method given in Example 21 using Hapten C.

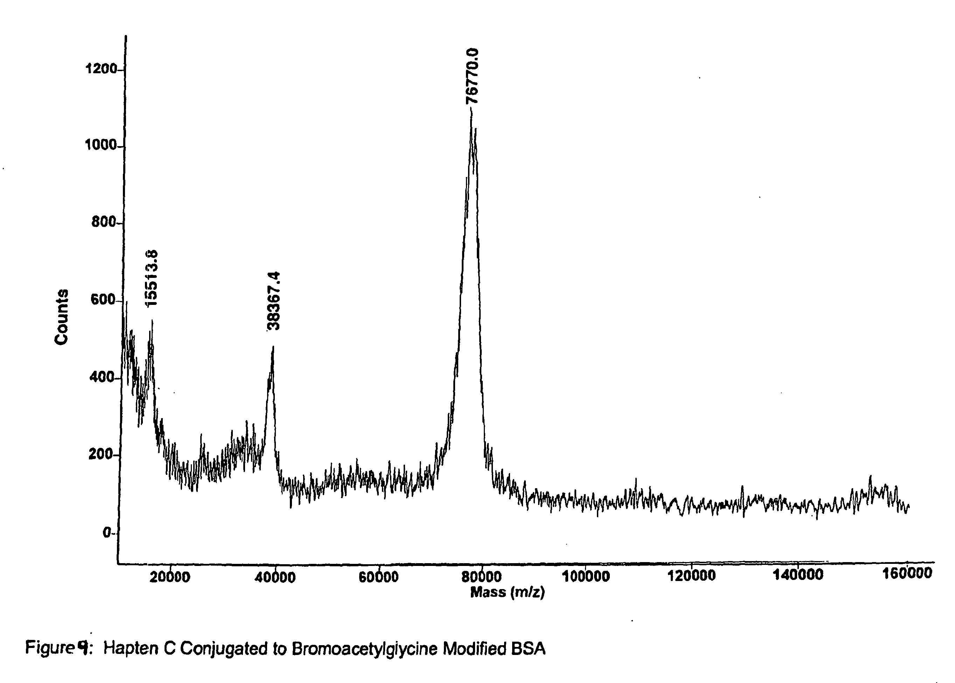

MALDI results (see Figure 9 of the accompanying drawings) showed 7.2 molecules of Hapten C had been conjugated to one molecule of modified BSA. Specifically, a major signal was present which indicates an average protonated mass for this sample of m/z 76,770. The signal at m/z 38,367 is consistent with the major component in doubly-charged form. A further signal was observed at m/z 15,514.

### EXAMPLE 23: Reaction Scheme 4

### Production of Immunogen F using BSA

50mg 1-ethyl-3-(3-dimethylaminopropyl)carbodiimide hydrochloride (EDC) was dissolved in 600µl water and immediately added to a solution of 100mg BSA (1.5µmol) in 4ml water. Hapten F (19.7mg, 45µmol) dissolved in 2ml anhydrous DMF was added dropwise, with stirring. 5mg sulfo-NHS was added and the resulting solution was incubated overnight at 37°C. The solution was then dialysed against 5L phosphate buffered saline (PBS), pH7.2, for 24 hours at 4°C, with stirring.

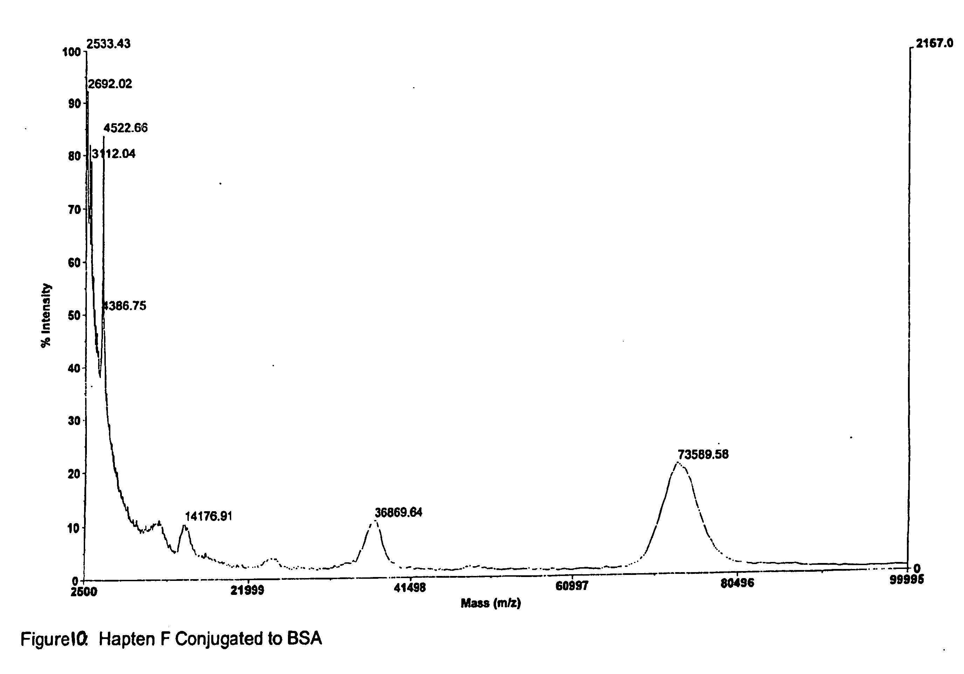

MALDI results (see Figure 10 of the accompanying drawings) showed 17.1 molecules of hapten F had been conjugated to one molecule of BSA. Specifically, a major signal was present which indicates an average protonated mass for this sample of m/z 73,590. The signal at m/z 36,870 is consistent with the major component in a doubly-charged form. Further signals were observed including that at m/z 14,177.

### EXAMPLE 24: Reaction Scheme 5

### Production of Immunogen G using BSA

This conjugation was performed by the method outlined in Example 23 using hapten G (12.57mg, 43µmol).

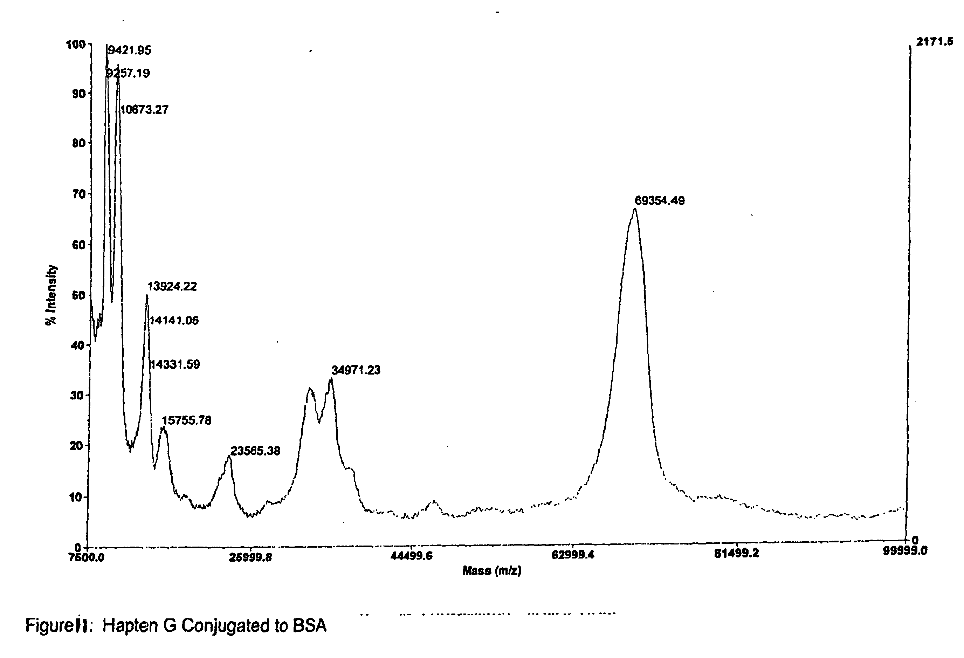

MALDI results (see Figure 11 of the accompanying drawings) showed 10.4 molecules of hapten G had been conjugated to one molecule of BSA. Specifically, a major signal was present which indicates an average protonated mass for this sample of m/z 69,355. The signal at m/z 34,971 is consistent with the major component in a doubly-charged form. Further signals were observed including that at m/z 13,924.

### EXAMPLE 25

### Conjugation of Haptens B and E to Labelling Agent (Horse Radish Peroxidase (HRP))

10mg of EDC was dissolved in 800µl water and immediately added to a solution of the hapten (1mg) in 200µl DMF. The resulting solution was mixed gently and then added to a solution of HRP (20mg) in 1ml water. After mixing, 5mg of sulfo-NHS was added and the entire reaction was incubated overnight at 37°C in the dark. The resulting conjugate was purified by passage through two PD10 columns (Pharmacia Biotech), eluted with 20mM PBS, pH 7.2, and then dialysed overnight at 4°C against 20mM PBS, pH 7.2.

### EXAMPLE 26

### Conjugation of Hapten G to Labelling Agent (HRP)

10µl triethylamine (TEA) was added to a solution of Hapten G (1mg) in 400µl DMF and the resulting solution was mixed for 10 minutes at room temperature. 4µl isobutylchloroformate (BCF) was then added and allowed to react for a further 10 minutes at room temperature. The activated hapten was immediately added to a solution of HRP (20mg) in 2ml water and the reaction was incubated overnight, with mixing, at room temperature in the dark. The resulting conjugate was purified by passage through two PD10 columns (Pharmacia Biotech), eluted with 20mM PBS, pH 7.2, and then dialysed overnight at 4°C against 20mM PBS, pH 7.2.

### EXAMPLE 27

### Immunisation and Bleeding

An aqueous solution of each of the immunogens prepared in Examples 21, 22, 23 and 24 was formulated with Freund's Complete Adjuvant (FCA) to form an emulsion consisting of 2mg/ml immunogen in 50% (v/v) FCA. Three sheep were immunized with this emulsion, 0.25ml being subcutaneously injected at each of four sites in the flank of each animal. Subsequent immunisations (boosts) contained 1mg/ml immunogen emulsified in 50%(v/v) Freund's Incomplete Adjuvant (FIA) and were administered in the same manner at monthly intervals for 1 year. Blood sampling took place 7 to 14 days after each boost. Each sample was processed to produce antiserum, which was further purified by caprylic acid and ammonium sulfate precipitation to yield an immunoglobulin G (IgG) fraction. The IgG fraction was evaluated by competitive ELISA microtiter plate assay, as described below.

### EXAMPLE 28

### Competitive ELISA microtiter plate assays for nor fentanyl and fentanyl.

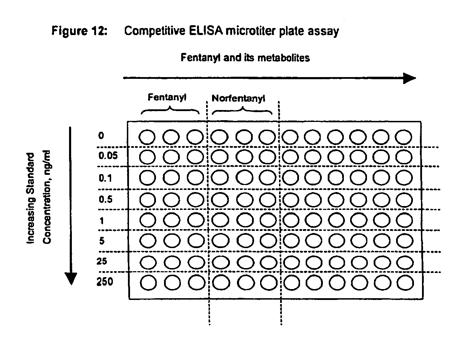

(a) The wells of an enhanced binding 96 well polystyrene microtiter plate were coated with the IgG fraction of the antiserum raised to Immunogen A (Hapten A-BSA) (Example 21), diluted in 10mM Tris, pH 8.5 (125µl/well). The appropriate antibody coating dilution was determined using standard ELISA chequerboard techniques. The plate was incubated for 2 hours at 37°C, washed 4 times with Tris buffered saline containing Tween 20 (TBST) and tapped dry. Standard solutions of fentanyl and norfentanyl were prepared in TBST at 0, 1, 5, 10, 50 and 500ng/ml and 25µl of each was added to the appropriate wells (see Figure 12). 100µl of conjugate B (hapten B-HRP) (Example 25), diluted in Tris buffer containing EDTA, D-mannitol, sucrose, thimerosal and BSA, was added to each of the wells, as shown in Figure 12. The appropriate dilution of conjugate was also determined using standard ELISA chequerboard techniques. The plate was incubated at 37°C for 2 hours. The excess unbound conjugate was removed by washing 6 times over a 10 minute period with TBST. 125µl of tetramethylbenzidine (TMB) substrate solution was added to each well of the plate, which was then incubated for 15 to 20 minutes in the dark at room temperature. The reaction was terminated by addition of 125µl 0.2M H₂SO₄ to each well. The absorbance was then measured at 450nm using a microtiter plate reader. The data generated in the assay is presented in Table 1 below.

**Table 1: Data generated from a competitive microtiter plate assay for norfentanyl and fentanyl employing antiserum raised to immunogen A (hapten A-BSA) (Example 21) and conjugate B (hapten B-HRP) as detection reagent (Example 25).**

| **Standard Concentration ng/ml** | **Fentanyl** | | **Norfentanyl** | |
|---|---|---|---|---|
| | *A₄₅₀* | %B/B₀ | *A₄₅₀* | %B/B₀ |
| 0 | **2.3** | | **2.29** | |
| 0.05 | **2.07** | | **2.18** | **94.94** |
| 0.1 | **1.84** | **80.2** | **2.11** | **91.83** |
| 0.5 | **1.26** | **54.91** | **1.94** | **84.57** |
| 1 | **0.87** | **38.01** | **1.77** | **77.27** |
| 5 | **0.27** | **11.74** | **1.37** | **59.8** |
| 25 | **0.08** | **3.68** | **0.76** | **33.32** |
| 250 | **0.08** | **3.53** | **0.24** | **10.57** |
| | | | | |
| **IC₅₀** | **0.61 ng/ml** | | **9.07 ng/ml** | |

| | | | | |
|---|---|---|---|---|
| *A₄₅₀* = absorbance at 450nm B = absorbance at 450nm at xng/ml standard concentration B₀ = absorbance at 450nm at 0ng/ml standard concentration IC₅₀ = standard concentration which produces 50% B/B₀ | | | | |

(b) In a similar manner to that described in Example 28(a) and using the same definitions for A₄₅₀, B, B₀ and IC₅₀, the wells of a 96 well microtiter plate were coated with the IgG fraction of the antiserum raised to immunogen A (hapten A-BSA) (Example 21) and conjugate G (hapten G-HRP) (Example 26) was employed as detection reagent. The data generated is presented in Table 2 below.

**Table 2: Data generated from a competitive microtiter plate assay for norfentanyl and fentanyl employing antiserum raised to immunogen A (hapten A-BSA) (Example 21) and conjugate G (hapten G-HRP) as detection reagent (Example 26).**

| **Standard Concentration ng/ml** | **Fentanyl** | | **Norfentanyl** | |
|---|---|---|---|---|
| | *A₄₅₀* | %B/B₀ | *A₄₅₀* | %B/B₀ |
| 0 | **1.99** | | **1.94** | |
| 0.05 | **1.94** | **97.09** | **1.85** | **95.69** |
| 0.1 | **1.94** | **97.54** | **1.82** | **93.93** |
| 0.5 | **1.71** | **85.9** | **1.67** | **86.31** |
| 1 | **1.47** | **73.66** | **1.52** | **78.48** |
| 5 | **0.4** | **19.97** | **1.03** | **53.04** |
| 25 | **0.07** | **3.54** | **0.56** | **29.14** |
| 250 | **0.03** | **1.43** | **0.14** | **7.05** |
| | | | | |
| **IC₅₀** | **2.03ng/ml** | | **6.14ng/ml** | |

(c) In a similar manner to that described in Example 28(a), the wells of a 96 well microtiter plate were coated with the IgG fraction of the antiserum raised to immunogen C (hapten C-BSA) (Example 22) and conjugate E (hapten E-HRP) (Example 25) was employed as detection reagent. The data generated is presented in Table 3 below.

**Table 3: Data generated from a competitive microtiter plate assay for norfentanyl and fentanyl employing antiserum raised to immunogen C (hapten C-BSA) (Example 22) and conjugate E (hapten E-HRP) as detection reagent (Example 25).**

| **Standard Concentration ng/ml** | **Fentanyl** | | **Norfentanyl** | |
|---|---|---|---|---|
| | *A₄₅₀* | %B/B₀ | *A₄₅₀* | %B/B₀ |
| 0 | **2.3** | | **2.2** | |
| 0.05 | **1.9** | **82.2** | **2.1** | **94.3** |
| 0.1 | **1.6** | **68.2** | **2.0** | **90.8** |
| 0.5 | **0.8** | **34.4** | **1.9** | **88.5** |
| 1 | **0.5** | **23.4** | **1.9** | **88.6** |
| 5 | **0.2** | **8.4** | **1.9** | **89.2** |
| 25 | **0.1** | **2.8** | **2.0** | **90.1** |
| 250 | **0.0** | **1.0** | **2.0** | **89.6** |
| | | | | |
| **IC₅₀** | **0.24ng/ml** | | **>250ng/ml** | |

### Bibliography

Camu et al; Anesth. Analg.; 61; pp657-61 (1982)
Frincke, J.M. & Henderson, G.L.; Drug Metab. Dispos.; 8(6); pp425-7 (1980).
Hammargren, W.R. & Henderson, G.L.; J. Anal. Toxicol.; 12; pp183-191 (1988).
Henderson et al; J. Pharmacol. Exp. Ther.; 192 (2); pp489-96 (1975).
Makowski et al; Annals of Clinical and Laboratory Science; 25 (2); pp169-177 (1995).
McDonald et al; Res. Commun. Chem. Pathol. Pharmacol.; 57 (3); pp389-407 (1987).
Meuldermans et al; Arch. Int. Pharmacodyn. Ther.; 257; pp4-19 (1982).
Michiels et al; Eur. J. Clin. Pharmacol.; 12 (2); pp153-8 (1977).
Ruangyuttikarn et al; J. Anal. Toxicol.; 14; pp160-164 (1990).
Silverstein et al; Anesthesia and Analgesia; 76; pp618-621 (1993).
Watts, V.W. & Caplan, Y. H.; J. Anal. Toxicol; 14; pp266-272 (1990).

## Claims

1. An immunogen of the formula: 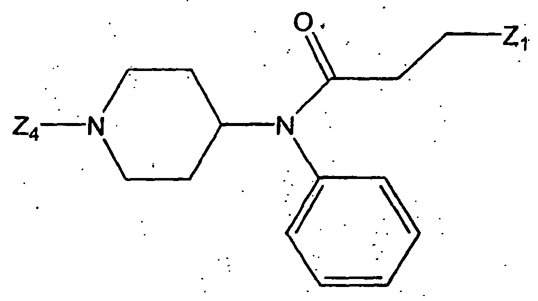 , in which Z₁ is hydrogen; and Z₄ is selected a crosslinker coupled to an antigenicity-conferring carrier material or 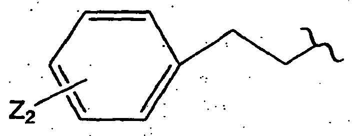 , in which Z₂ is a crosslinker coupled to an antigenicity-conferring carrier material at an ortho, a meta or a para position,
with the proviso that the immunogen is not a heterodimer of the formula: (BKAn)(X)(Y); where BKAn is a bradykinin antagonist peptide;
wherein Y is : 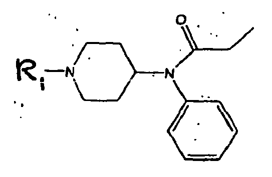 where R₁ is the linking group X of the formula CH₂CH₂(Phe)CH₂C(O).

2. A conjugate of the formula: 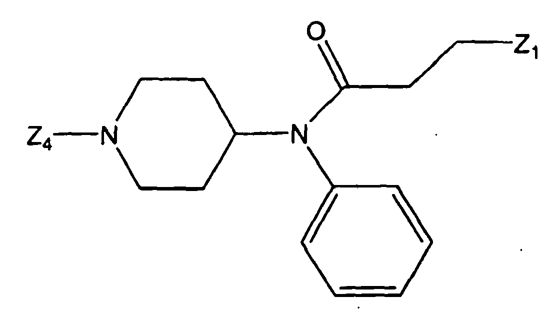 , in which Z₁ is hydrogen; Z₄ is selected from a crosslinker covalently bonded to a labelling agent which is detectable or 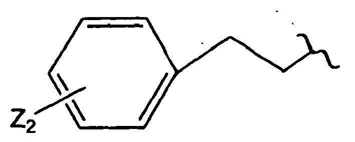 in which Z₂ is a crosslinker covalently bonded to a labelling agent which is detectable at an ortho, a meta or a para position.

3. An immunogen or a conjugate as claimed in Claim 1 or 2, in which the crosslinker of Z₂ is at the para position.

4. An immunogen or a conjugate as claimed in any one of the preceding claims, wherein the crosslinker terminates, at its free end prior to coupling to the antigenicity-conferring carrier material or bonding to a labelling agent to form the immunogen or the conjugate, respectively, with -CO-R, in which R is hydroxyl and a short chain alkyl (C₁₋₅), preferably a (C₁₋₂) alkyl, moiety.

5. An immunogen or a conjugate as claimed in any one of Claims 1 to 3, in which the crosslinker, prior to coupling to the antigenicity-conferring carrier material or bonding to a labelling agent to form the immunogen or the conjugate, respectively, comprises:
-(NH)ₐ-(CO)_{b}-X_{c}-(CH₂)_{d}-Sₑ-CO-R
wherein, independently, a is 0 or 1; b is 0 or 1; X is oxygen or sulfur; c is 0 or 1; d is selected from the integers 1-5; e is 0 or 1 and R is a short chain alkyl (C₁₋₅) moiety, preferably a C₁₋₂ alkyl moiety, or a hydroxyl moiety.

6. An immunogen or a conjugate as claimed in Claim 5, in which a is 1; b is 1; c is 0; and d is 2.

7. An immunogen or a conjugate as claimed in Claim 6, in which e is 1; and R is methyl.

8. An immunogen or a conjugate as claimed in Claim 6, in which e is 0; and R is hydroxyl.

9. An immunogen or a conjugate as claimed in any one of Claims 6-8, in which Z₁ is hydrogen and the Z₂ crosslinker is at the para position.

10. An immunogen or a conjugate as claimed in Claim 5, wherein a is 0; b is 0; and c is 0.

11. An immunogen or a conjugate as claimed in Claim 10, wherein d is 0; e is 1 and R is methyl.

12. An immunogen or a conjugate as claimed in Claim 10, wherein d is 0 or 1; e is 0; and R is hydroxyl.

13. An immunogen or conjugate as claimed in any one of Claims 10-12, wherein Z₄ is the crosslinker.

14. An immunogen as claimed in any one of Claims 1 and 3 to 13, in which the antigenicity-conferring carrier material is selected from a protein, a protein fragment, a synthetic polypeptide and a semi-synthetic polypeptide.

15. Antibodies raised against an immunogen selected from the group comprising an immunogen of the formula: 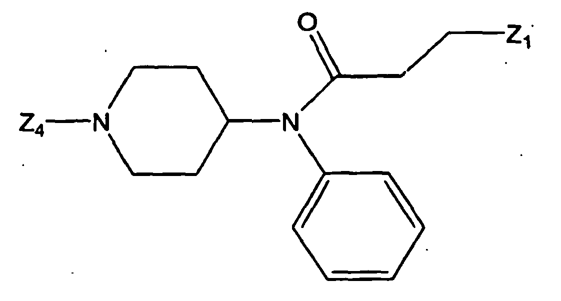 , in which Z₁ is hydrogen; and Z₄ is selected a crosslinker coupled to an antigenicity-conferring carrier material or 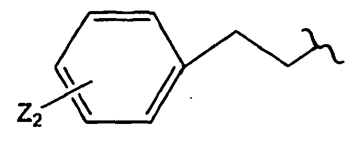 , in which Z₂ is a crosslinker coupled to an antigenicity-conferring carrier material at an ortho, a meta or a para position or an immunogen as claimed in any one of Claims 3 to 14, wherein the antibodies are capable of binding with at least one structural epitope of a metabolite of fentanyl or of a metabolite of a fentanyl analog.

16. A process of preparing the antibodies as claimed in Claim 15, the process comprising the steps of immunising an animal by repeated administration of the immunogen selected from the group comprising the immunogen of the formula: 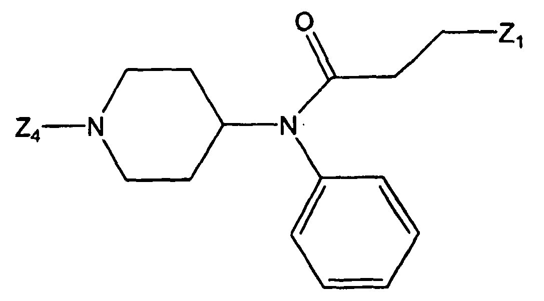 , in which Z₁ is hydrogen; and Z₄ is selected a crosslinker coupled to an antigenicity-conferring carrier material or 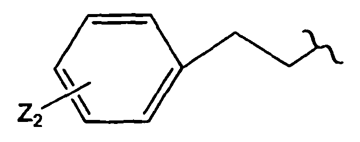 , in which Z₂ is a crosslinker coupled to an antigenicity-conferring carrier material at an ortho, a meta or a para position or an immunogen of any one of Claims 3 to 14, and collecting the resulting serum from the immunised animal.

17. A conjugate as claimed in any one of Claims 2 to 13, in which the detectable labelling agent is selected from an enzyme, which is preferably a peroxidase, most preferably a horseradish peroxidase; a luminescent substance, which is preferably selected from a bioluminescent substance, a chemiluminescent substance and a fluorescent substance, or a mixture thereof; and a radioactive substance; or a mixture thereof.

18. A method for detecting, or determining the quantity of, metabolites of fentanyl, or of metabolites of fentanyl analogs in a sample, the method comprising contacting the sample with a conjugate as claimed in any one of Claims 2 to 13 and 17, or a mixture thereof and with the antibodies as claimed in Claim 15 or a mixture thereof; detecting, or determining the quantity of, bound conjugate; and deducing from a calibration curve the presence, or the amount of, metabolites of fentanyl, and metabolites of fentanyl analogs in the sample.

19. A kit for detecting, or determining the quantity of, metabolites of fentanyl, or metabolites of fentanyl analogs, the kit including a conjugate as claimed in any one of Claims 2 to 13 and 17 or a mixture thereof and the antibodies as claimed in Claim 15 or a mixture thereof.

20. A method or a kit according to Claim 18 or 19, in which the antibodies are raised against an immunogen in which Z₂ is the crosslinker.

21. A method or a kit according to any one of Claims 18 to 20, in which Z₄ of the conjugate is the crosslinker.

## Patentansprüche

1. Immunogen der Formel: 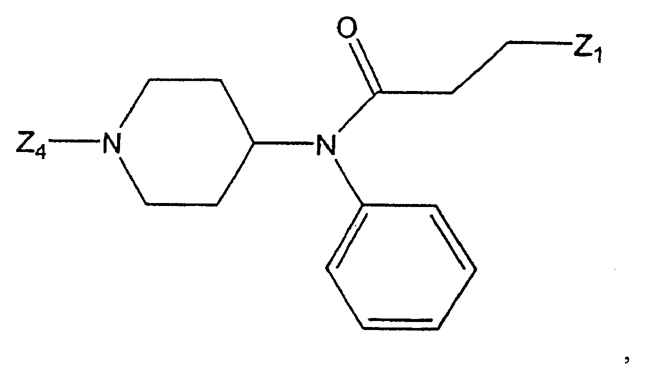 worin Z₁ Wasserstoff ist und Z₄ aus einem Crosslinker ausgewählt ist, der an ein Antigenität-verleihendes Trägermaterial gekoppelt ist, oder 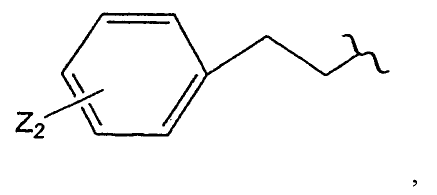 worin Z₂ ein Crosslinker ist, der an ein Antigenität-verleihendes Trägermaterial an einer ortho-, einer meta- oder einer para-Position gekoppelt ist,
mit der Maßgabe, dass das Immunogen kein Heterodimer der Formel (BKAn)(X)(Y) ist;
worin BKAn ein Bradykinin-Antagonist-Peptid ist;
worin Y Folgendes ist: 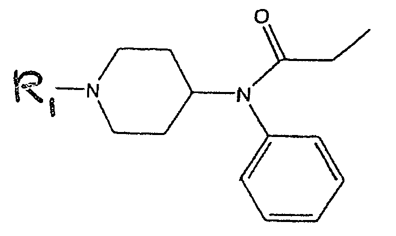 worin R₁ die verbindende Gruppe X der Formel CH₂CH₂(Phe)CH₂C(O) ist.

2. Konjugat der Formel: 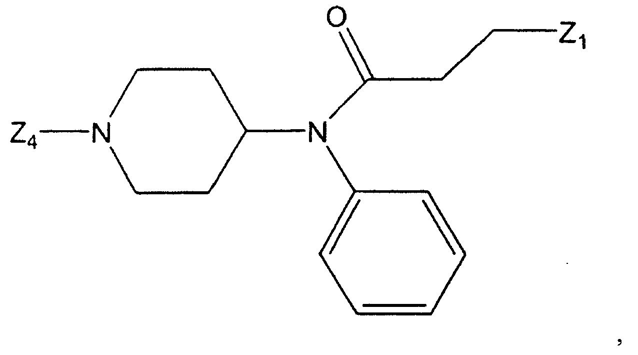 worin Z₁ Wasserstoff ist; Z₄ aus einem Crosslinker ausgewählt ist, der kovalent an ein Markierungsagens, das detektierbar ist, gebunden ist, oder 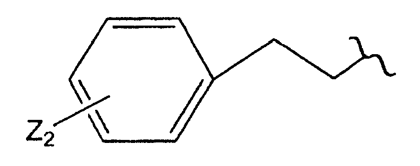 worin Z₂ ein Crosslinker ist, der kovalent an ein Markierungsagens gebunden ist, das an einer ortho-, einer meta- oder einer para-Position detektierbar ist.

3. Immunogen oder ein Konjugat nach Anspruch 1 oder 2, in dem der Crosslinker von Z₂ an der para-Position ist.

4. Immunogen oder ein Konjugat nach einem der vorstehenden Ansprüche, worin der Crosslinker an seinem freien Ende vor dem Koppeln an das Antigenität-verleihende Trägermaterial oder Binden an ein Markierungsagens zur Bildung des Immunogens bzw. des Konjugats mit -CO-R endet, worin R Hydroxyl und ein kurzkettiges Alkyl (C₁₋₅), bevorzugt eine (C₁₋₂)-Alkyl-Einheit ist.

5. Immunogen oder ein Konjugat nach einem der Ansprüche 1 bis 3, worin der Crosslinker vor dem Koppeln an das Antigenität-verleihende Trägermaterial oder Binden an ein Markierungsagens zur Bildung des Immunogens bzw. des Konjugats Folgendes umfasst:
-(NH)ₐ-(CO)_{b}-X_{c}-(CH₂)_{d}-Sₑ-CO-R
worin unabhängig a 0 oder 1 ist; b 0 oder 1 ist; X Sauerstoff oder Schwefel ist; c 0 oder 1 ist; d aus den ganzen Zahlen 1-5 ausgewählt ist; e 0 oder 1 ist und R eine kurzkettige (C₁₋₅)-Alkyl-Einheit, bevorzugt eine C₁₋₂-Alkyl-Einheit oder eine Hydroxyl-Einheit ist.

6. Immunogen oder ein Konjugat nach Anspruch 5, worin a 1 ist; b 1 ist; c 0 ist und d 2 ist.

7. Immunogen oder ein Konjugat nach Anspruch 6, worin e 1 ist und R Methyl ist.

8. Immunogen oder ein Konjugat nach Anspruch 6, worin e 0 ist und R Hydroxyl ist.

9. Immunogen oder ein Konjugat nach einem der Ansprüche 6-8, worin Z₁ Wasserstoff ist und der Z₂-Crosslinker an der para-Position ist.

10. Immunogen oder ein Konjugat nach Anspruch 5, worin a 0 ist; b 0 ist und c 0 ist.

11. Immunogen oder ein Konjugat nach Anspruch 10, worin d 0 ist; e 1 ist und R Methyl ist.

12. Immunogen oder ein Konjugat nach Anspruch 10, worin d 0 oder 1 ist; e 0 ist und R Hydroxyl ist.

13. Immunogen oder Konjugat nach einem der Ansprüche 10-12, worin Z₄ der Crosslinker ist.

14. Immunogen nach einem der Ansprüche 1 und 3 bis 13, worin das Antigenität-verleihende Trägermaterial aus einem Protein, einem Proteinfragment, einem synthetischen Polypeptid und einem halbsynthetischen Polypeptid ausgewählt ist.

15. Antikörper, die gegen ein Immunogen gebildet sind, das aus der Gruppe ausgewählt ist, die ein Immunogen der folgenden Formel umfasst: 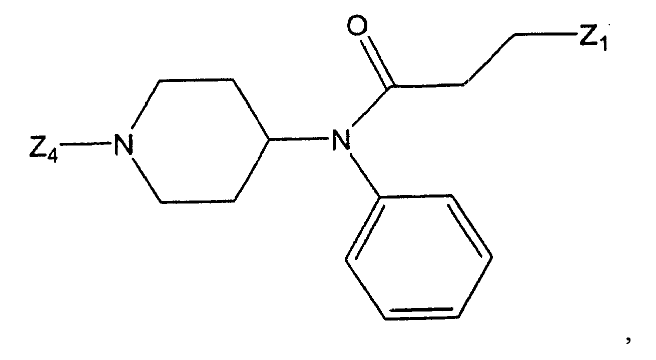 worin Z₁ Wasserstoff ist und Z₄ aus einem Crosslinker ausgewählt ist, der an ein Antigenität-verleihendes Trägermaterial gekoppelt ist, oder 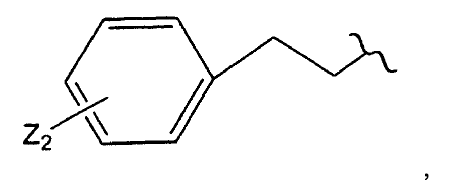 worin Z₂ ein Crosslinker ist, der an ein Antigenität-verleihendes Trägermaterial an einer ortho-, einer meta- oder einer para-Position gekoppelt ist, oder gegen ein Immunogen nach einem der Ansprüche 3 bis 14, worin die Antikörper fähig sind, mit mindestens einem strukturellen Epitop eines Metaboliten von Fentanyl oder eines Metaboliten eines Fentanyl-Analogons zu binden.

16. Verfahren zur Herstellung der Antikörper nach Anspruch 15, wobei das Verfahren die folgenden Schritte umfasst: des Immunisierens eines Tieres durch wiederholte Verabreichung des Immunogens, das aus der Gruppe ausgewählt ist, die das Immunogen der folgenden Formel umfasst: 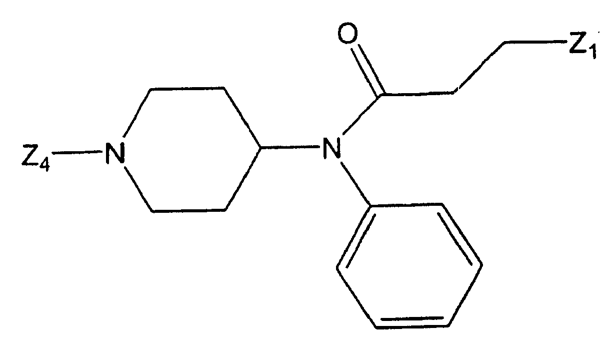 worin Z₁ Wasserstoff ist und Z₄ aus einem Crosslinker ausgewählt ist, der an ein Antigenität-verleihendes Trägermaterial gekoppelt ist, oder 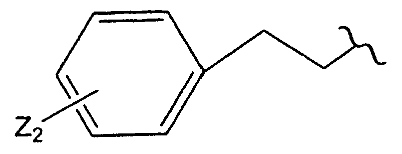 worin Z₂ ein Crosslinker ist, der an ein Antigenität-verleihendes Trägermaterial an einer ortho-, einer meta- oder einer para-Position gekoppelt ist, oder eines Immunogens nach einem der Ansprüche 3 bis 14; und des Sammelns des entstehenden Serums aus dem immunisierten Tier.

17. Konjugat nach einem der Ansprüche 2 bis 13, worin das detektierbare Markierungsagens aus Folgenden ausgewählt ist: einem Enzym, das bevorzugt eine Peroxidase, am bevorzugtesten eine Meerrettichperoxidase ist; einer lumineszierenden Substanz, die bevorzugt aus einer biolumineszierenden Substanz, einer chemilumineszierenden Substanz und einer fluoreszierenden Substanz oder einer Mischung davon ausgewählt ist; und einer radioaktiven Substanz; oder einer Mischung davon.

18. Verfahren zum Detektieren oder Bestimmen der Quantität von Metaboliten von Fentanyl oder von Metaboliten von Fentanyl-Analoga in einer Probe, wobei das Verfahren Folgendes umfasst: Inkontaktbringen der Probe mit einem Konjugat nach einem der Ansprüche 2 bis 13 und 17 oder einer Mischung davon und mit den Antikörpern nach Anspruch 15 oder einer Mischung davon; Detektieren oder Bestimmen der Quantität von gebundenem Konjugat; und Ableiten der Gegenwart oder der Menge von Metaboliten von Fentanyl und Metaboliten von Fentanyl-Analoga in der Probe aus einer Kalibrierungskurve.

19. Kit zum Detektieren oder Bestimmen der Quantität von Metaboliten von Fentanyl oder Metaboliten von Fentanyl-Analoga, wobei der Kit ein Kojugat nach einem der Ansprüche 2 bis 13 und 17 oder eine Mischung davon und die Antikörper nach Anspruch 15 oder ein Mischung davon einschließt.

20. Verfahren oder ein Kit gemäß Anspruch 18 oder 19, worin die Antikörper gegen ein Immunogen, in dem Z₂ der Crosslinker ist, gebildet sind.

21. Verfahren oder ein Kit gemäß einem der Ansprüche 18 bis 20, worin Z₄ des Konjugats der Crosslinker ist.

## Revendications

1. Immunogène de formule : 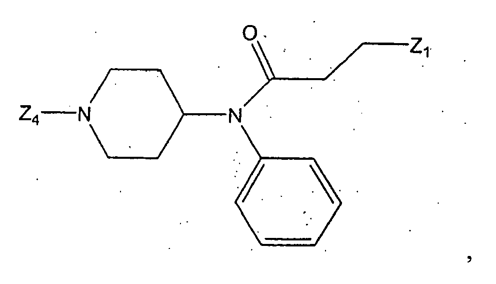 dans laquelle Z₁ est hydrogène ; et Z₄ est choisi parmi un agent de réticulation couplé à une substance support conférant une antigénicité ou 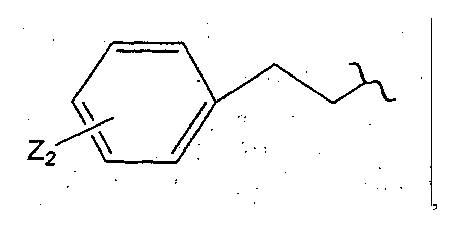 dans laquelle Z₂ est un agent de réticulation couplé à une substance support conférant une antigénicité à une position ortho, méta ou para,
à condition que l'immunogène ne soit pas un hétérodimère de formule :
(BKAn)(X)(Y) ;
où BKAn est un peptide antagoniste de la bradykinine ;
où Y est : 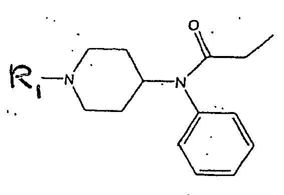 où R₁ est le groupe de liaison X de formule CH₂CH₂(Phe)CH₂C(O).

2. Conjugué de formule : 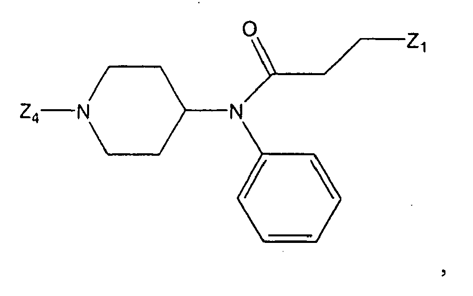 dans laquelle Z₁ est hydrogène ; Z₄ est choisi parmi un agent de réticulation lié de manière covalente à un agent de marquage qui peut être détecté ou 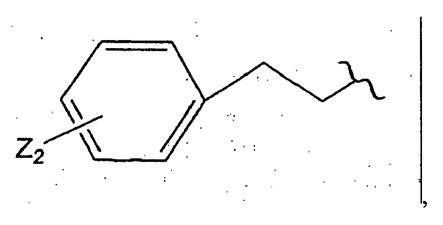 dans laquelle Z₂ est un agent de réticulation lié de manière covalente à un agent de marquage qui peut être détecté à une position ortho, méta ou para.

3. Immunogène ou conjugué selon la revendication 1 ou 2, dans lequel l'agent de réticulation de Z₂ se trouve à la position para.

4. Immunogène ou conjugué selon l'une quelconque des revendications précédentes, dans lequel l'agent de réticulation se termine, à son extrémité libre avant le couplage à la substance support conférant une antigénicité ou la liaison à un agent de marquage pour former l'immunogène ou le conjugué, respectivement, par -CO-R, où R est un hydroxyle et un motif alkyle en (C₁₋₅) à chaîne courte, de préférence un motif alkyle en (C₁₋₂).

5. Immunogène ou conjugué selon l'une quelconque des revendications 1 à 3, dans lequel l'agent de réticulation, avant le couplage à la substance support conférant une antigénicité ou la liaison à un agent de marquage pour former l'immunogène ou le conjugué, respectivement, comprend :
-(NH)ₐ-(CO)_{b}-X_{c}-(CH₂)_{d}-Sₑ-CO-R
où, de façon indépendante, a vaut 0 ou 1, b vaut 0 ou 1, X est oxygène ou soufre ; c vaut 0 ou 1, d est choisi parmi les entiers 1-5 ; e vaut 0 ou 1 et R est un motif alkyle en (C₁₋₅) à chaîne courte, de préférence un motif alkyle en C₁₋₂, ou un motif hydroxyle.

6. Immunogène ou conjugué selon la revendication 5, dans lequel a vaut 1 ; b vaut 1 ; c vaut 0 ; et d vaut 2.

7. Immunogène ou conjugué selon la revendication 6, dans lequel e vaut 1 ; et R est méthyle.

8. Immunogène ou conjugué selon la revendication 6, dans lequel e vaut 0 ; et R est hydroxyle.

9. Immunogène ou conjugué selon l'une quelconque des revendications 6-8, dans lequel Z₁ est hydrogène et l'agent de réticulation Z₂ se trouve à la position para.

10. Immunogène ou conjugué selon la revendication 5, dans lequel a vaut 0 ; b vaut 0; et c vaut 0.

11. Immunogène ou conjugué selon la revendication 10, dans lequel d vaut 0 ; e vaut 1 ; et R est méthyle.

12. Immunogène ou conjugué selon la revendication 10, dans lequel d vaut 0 ou 1 ; e vaut 0 ; et R est hydroxyle.

13. Immunogène ou conjugué selon l'une quelconque des revendications 10-12, dans lequel Z₄ est l'agent de réticulation.

14. Immunogène selon l'une quelconque des revendications 1 et 3 à 13, dans lequel la substance support conférant une antigénicité est choisie parmi une protéine, un fragment protéique, un polypeptide synthétique et un polypeptide semi-synthétique.

15. Anticorps dirigés contre un immunogène choisi parmi le groupe comprenant un immunogène de formule : 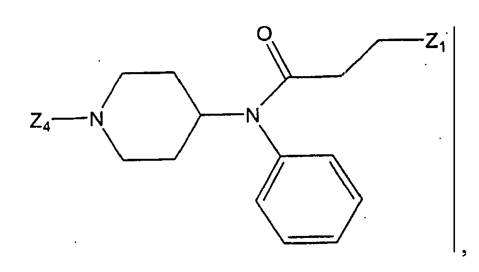 dans laquelle Z₁ est hydrogène ; et Z₄ est choisi parmi un agent de réticulation couplé à une substance support conférant une antigénicité ou 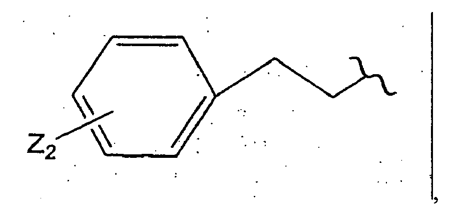 dans laquelle Z₂ est un agent de réticulation couplé à une substance support conférant une antigénicité à une position ortho, méta ou para, ou un immunogène selon l'une quelconque des revendications 3 à 14, où les anticorps sont capables de se lier avec au moins un épitope structural d'un métabolite de fentanyle ou d'un métabolite d'un analogue de fentanyle.

16. Procédé de préparation des anticorps selon la revendication 15, le procédé comprenant les étapes consistant à immuniser un animal par l'administration répétée de l'immunogène choisi parmi le groupe comprenant l'immunogène de formule : 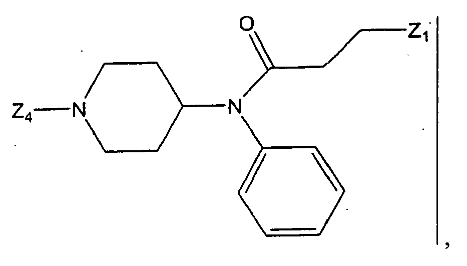 dans laquelle Z₁ est hydrogène ; et Z₄ est choisi parmi un agent de réticulation couplé à une substance support conférant une antigénicité ou 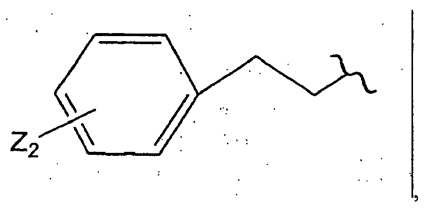 dans laquelle Z₂ est un agent de réticulation couplé à une substance support conférant une antigénicité à une position ortho, méta ou para, ou un immunogène selon l'une quelconque des revendications 3 à 14, et à récupérer le sérum résultant de l'animal immunisé.

17. Conjugué selon l'une quelconque des revendications 2 à 13, dans lequel l'agent de marquage qui peut être détecté est choisi parmi une enzyme, qui est de préférence une peroxydase, de façon tout à fait préférable une peroxydase de raifort ; une substance luminescente, qui est de préférence choisie parmi une substance bioluminescente, une substance chimioluminescente et une substance fluorescente, ou un mélange de celles-ci ; et une substance radioactive ; ou un mélange de ceux-ci.

18. Méthode de détection ou de détermination de la quantité de métabolites de fentanyle ou de métabolites d'analogues de fentanyle dans un échantillon, la méthode comprenant la mise en contact de l'échantillon avec un conjugué selon l'une quelconque des revendications 2 à 13 et 17, ou un mélange de ceux-ci, et avec les anticorps selon la revendication 15, ou un mélange de ceux-ci ; la détection ou la détermination de la quantité de conjugué lié ; et la déduction, à partir d'une courbe d'étalonnage, de la présence ou de la quantité de métabolites de fentanyle et de métabolites d'analogues de fentanyle dans l'échantillon.

19. Kit de détection ou de détermination de la quantité de métabolites de fentanyle ou de métabolites d'analogues de fentanyle, le kit comportant un conjugué selon l'une quelconque des revendications 2 à 13 et 17, ou un mélange de ceux-ci, et les anticorps selon la revendication 15, ou un mélange de ceux-ci.

20. Méthode ou kit selon la revendication 18 ou 19, dans lequel/laquelle les anticorps sont dirigés contre un immunogène dans lequel Z₂ est l'agent de réticulation.

21. Méthode ou kit selon l'une quelconque des revendications 18 à 20, dans lequel/laquelle Z₄ du conjugué est l'agent de réticulation.
